# EUROPEAN PATENT APPLICATION

(11) **EP 0 753 581 A1**
(43) Date of publication of application: **15.01.1997**
(21) Application number: 95110727.5
(22) Date of filing: 10.07.1995
(51) Int. Cl.: C12N 15/86, C12N 7/01, C12N 7/04, A61K 38/19, A61K 39/21, A61K 39/275, A61K 39/29

(54) **Improved recombinant eukaryotic cytoplasmic viruses, method for their production and their use as vaccines**

(71) Applicant: IMMUNO AG, 1221 Wien (AT)
(72) Inventor: Scheiflinger, Friedrich, Dr., A-1190 Wien (AT); Antoine, Gerhard, Dr., A-2301 Gr.Enzersdorf (AT); Falkner, Falko-Günter, Dr., A-2304 Orth/Donau (AT); Dorner, Friedrich, Prof. Dr., A-1230 Wien (AT); Eibl, Johan, Dr., AT-1180 Wien (AT)
(74) Representative: Kolb, Helga, Dr. Dipl.-Chem.

(57) **Abstract**

The invention relates to a method for producing a recombinant eukaryotic cytoplasmic DNA virus, comprising the steps of inserting a heterologous DNA construct into a eukaryotic cytoplasmic DNA virus genome, wherein said heterologous DNA construct comprises one or more segments of foreign DNA and a double selection marker cassette which is flanked by at least two direct repeat DNA sequences wherein said double selection marker cassette comprises a dominant selection marker gene and a color selection marker gene, and wherein at least one foreign DNA segment does not occur within the DNA sequence bound by said direct repeat sequences and transfecting cells with said eukaryotic cytoplasmic DNA virus containing said heterologous DNA construct and selecting a recombinant eukaryotic cytoplasmic DNA virus that does not contain said double selection marker cassette.

Furthermore, the invention relates to DNA molecules for said method as well as recombinant viruses produced by said method and vaccines comprising said viruses.

## Description

### Background of the Invention

Recombinant eukaryotic cytoplasmic DNA viruses, such as poxviruses and iridoviruses, have been used as vectors for expression of heterologous genes, and the potential use of these viruses as live vaccines has been previously evaluated. See, for example, Moss, *Science* 252: 1662-1667 (1991), and Paoletti, PCT application WO 89/03429.

The major methods for producing recombinant eukaryotic cytoplasmic DNA viruses are via techniques involving homologous *in vivo* recombination (Panicali, D. and Paoletti, E. Proc. Natl. Acad. Sci. USA 79: 4927-4931 (1982); Mackett *et al*., Proc. Natl. Acad. Sci. USA 79:7415-7419 (1982)) and direct molecular cloning (Scheiflinger *et al*., Proc. Natl. Acad. Sci. USA 89: 9977-9981 (1992); Merchlinsky, M & Moss, B. Virol. 190: 522-526 (1992)). Both of these methods produce an initial mixture of wild-type and recombinant virus, and a selection method is therefore required to distinguish between the wild-type virus, which predominates in the mixture, and the relatively rare recombinant virus.

Several methods are currently used to facilitate this screening. One common approach is to produce the recombinant virus by inserting the heterologous gene, *via* homologous recombination, into the thymidine kinase (*tk*) gene of the virus, thereby causing loss of viral thymidine kinase activity. Virus-infected cells are then treated with bromodeoxyuridine, selectively killing wild-type virus and allowing selection of recombinant virus. A major problem of this approach is that it is limited to the use of thymidine kinase deficient (tk-negative) host cells and therefore cannot be used in chicken embryo fibroblast (CEF) cells, which are a preferred host cell for many attenuated strains of vaccinia or fowlpox. Inactivation of the *tk* locus is also associated with a severe attenuation of viral virulence, which is undesirable when, as is common for strains used in vaccine studies, the virus to be modified is already highly attenuated. Buller et al., Nature 317:813-815 (1985). A particularly attractive strain of vaccinia for vaccine purposes is the strain designated "MVA" short for "Modified Vaccinia Ankara," which has been used in large vaccine trials in Germany. Stickl et al., Dtsch. Med. Wschr. 99: 2386-2392 (1974); Mayr et al., Zbl. Bakt. Hyg., I. Abt. Orig. B 167: 375-390 (1978). An additional problem with the homologous recombination method is the relatively high background of spontaneous, non-recombinant, *tk*-negative mutants observed by this screening method. Use of bromodeoxyuridine selection obviously requires that the *tk* locus be used for insertion of the heterologous DNA.

A second selection method is the use of color marker screening, whereby the DNA construct containing the desired heterologous gene also contains a gene encoding a color-indicating enzyme, such as β-galactosidase (β-gal). Cells infected with the recombinant virus are then selected by addition of the substrate for the enzyme and selecting by color change. See Chakrabarti et al., Mol. Cell. Biol. 5: 3403-3409 (1985). This method is typically used as an auxiliary means of discerning true recombinant viruses from false positives in the thymidine kinase insertion method described above, although in principle it can be used to detect recombinant viruses containing inserts anywhere within the viral genome. A major problem with this approach is that it requires the stable integration of a functional color-indicating gene within the viral genome, which is undesirable in many instances, especially where the recombinant virus is intended for vaccine purposes. A variant of this method involves using a host strain in which the *tk* locus is already disrupted by a functional β-gal gene, which is itself then disrupted by recombination, wherein recombinant viruses are identified by the absence of a color change on addition of a substrate for β-*gal*. This method, of course, suffers from the inherent problems associated with *tk*-negative viruses, as discussed above.

A third selection method is analogous to color-selection method described above, but uses a drug selection gene in place of the β-*gal* gene. Drug selection genes used in this method include the E. coli guanine phosphoribosyl transferase (*gpt*) gene (Falkner et al., J. Virol. 62: 1849-1854 (1988)), and hygromycin phosphotransferase (hph) (Zhou et al., Gene 107: 307-312 (1991)). A drawback with this method is that, as with the introduction of the color-selectable gene, the selection gene becomes stably integrated into the viral genome.

In a variation of this approach, known as transient dominant selection, the marker gene is not retained within the final recombinant virus. See Falkner et al., J. Virol. 64: 3108-3111 (1990). This method, however, is unsuitable for constructing chimeras derived from the highly attenuated strains of vaccinia or fowlpox virus preferred for vaccine use. These latter strains have a narrow host range, and, as noted above, are usually propagated in CEF cells. These cells tend to give irreproducible results with dominant marker selection, particularly with respect to formation of false-positive plaques.

It is apparent, therefore, that an improved method of producing recombinant eukaryotic cytoplasmic DNA viruses, that allows efficient selection of recombinant virus without resulting in stable integration of a marker gene into the virus is to be greatly desired. In particular, it is desirable that such a method should be applicable to use in preparing recombinant eukaryotic cytoplasmic DNA viruses based on attenuated parent strains of virus that are suitable for use in vaccines.

### Summary of the Invention

It is therefore an object of the present invention to provide a method for producing recombinant eukaryotic cytoplasmic DNA viruses that allows efficient selection of recombinant virus from a background of wild-type virus.

It is also an object of the present invention to provide a method for efficiently preparing recombinant eukaryotic cytoplasmic DNA viruses from highly attenuated virus strains.

It is a further object of the present invention to provide DNA molecules, preferably vector DNA molecules, for the construction of the recombinant eukaryotic cytoplasmic DNA viruses according to the method of the present invention.

It is a further object of the present invention to provide recombinant eukaryotic cytoplasmic DNA viruses from highly attenuated virus strains that can be propagated in *tk*-positive cells.

It is still a further object of the present invention to provide a vaccine employing the recombinant eukaryotic cytoplasmic DNA viruses along with a pharmaceutically acceptable carrier.

It is yet another object of the present invention to provide recombinant eukaryotic cytoplasmic DNA viruses that express at least one antigen of a pathogen and at least one immunomodulator, such as an interleukin.

It is still a further object of the present invention to provide a method of immunizing patients using the vaccines of the present invention.

In achieving these objects, there has been provided, in accordance with one aspect of the present invention, a DNA molecule comprising a multiple-cloning site and a double selection marker cassette which is flanked by at least two direct repeat DNA sequences, wherein the multiple-cloning site does not occur within the DNA sequence bound by said direct repeat DNA sequences, and wherein said double selection marker cassette comprises a dominant selection marker gene and a color selection marker gene.

In a preferred embodiment, said DNA molecule is a plasmid selected from pvA(1), pvA(2), pHA-vA(2)a, pTK-vA, pTKm-vA(1), pTKm-vA(2).

In accordance with another aspect of the present invention, a DNA molecule, preferably a vector DNA molecule, comprising one or more segments of foreign DNA, and a double selection marker cassette which is flanked by at least two direct repeat DNA sequences, wherein at least one foreign DNA segment does not occur within the DNA sequence bound by said direct repeat DNA sequences, and wherein said double selection marker cassette comprises a dominant selection marker gene and a color selection marker gene.

In a preferred embodiment, the foreign DNA segment comprises a gene encoding an antigen of a pathogen. Preferably, the antigen gene is from a pathogen selected from the group consisting of human hepatitis B virus, HIV, and tick borne encephalitis virus. More preferably the antigen gene is selected from the group consisting of HIV *env*, HIV *gag*, HIV *gagpol*, HIV *nef*, HBV preS1-S2-S-surface antigen gene, HBV pre-S2-S-surface antigen gene, HBV S-surface antigen gene, tick borne encephalitis *pre*M gene and tick borne encephalitis *E* gene.

In yet another preferred embodiment, the foreign DNA segment further comprises a gene encoding an immunomodulator. The immunomodulator is preferably a cytokine, wherein said cytokine is preferably interleukin 6 or interferon-γ.

In still another preferred embodiment, the color selection marker gene is the *E*. *coli lacZ* gene and the dominant selection marker gene is either the *E. coli hph* gene or the *E.coli gpt* gene.

In a still further embodiment of the invention, the foreign DNA comprises a functional fowlpox thymidine kinase gene.

In accordance with another aspect of the present invention, there has been provided a method for producing a recombinant eukaryotic cytoplasmic DNA virus, comprising the steps of inserting a heterologous DNA construct into a eukaryotic cytoplasmic DNA virus genome, wherein said heterologous DNA construct comprises one or more segments of foreign DNA, and a double selection marker cassette which is flanked by at least two direct repeat DNA sequences, wherein said double selection marker cassette comprises a dominant selection marker gene and a color selection marker gene, and wherein at least one foreign DNA segment does not occur within the DNA sequence bound by the direct repeat DNA sequences and transfecting cells with said eukaryotic DNA virus containing said heterologous DNA construct and selecting a cytoplasmic DNA virus that does not contain said double color selection marker cassette.

In another preferred embodiment of the method, the recombinant eukaryotic cytoplasmic DNA virus is a recombinant poxvirus virus, preferably a recombinant vaccinia virus or a recombinant avipoxvirus.

In another preferred embodiment of the method, the vaccinia virus is derived from a vaccinia MVA strain and the avipoxvirus is derived from a fowlpox strain.

In another preferred embodiment of the method, the inserting step is performed by *in vivo* homologous recombination or by direct molecular cloning.

In yet other preferred embodiments of the method, the color selection marker gene is the *E. coli lacZ* gene and the dominant selection marker gene is either the *E. coli gpt* gene or the *E. coli hph* gene.

In still other preferred embodiments of the method, the foreign DNA encodes an antigen of a pathogen preferably selected from the group consisting of human immunodeficiency virus, human hepatitis virus and encephalitis virus. In another preferred embodiment of the method, said antigen gene is selected from the group consisting of HIV env, HIV gag, HIV *gagpol*, HIV *nef*, HBV preS1-S2-S-surface antigen gene, HBV pre-S2-S-surface antigen gene, HBV S-surface antigen gene, tick borne encephalitis *preM* gene and tick borne encephalitis *E* gene. Non-viral antigens of interest include tumor antigens, antigens of allergens and antigens of bacteria and parasites.

In still other preferred embodiments of the method, said foreign DNA further comprises a gene encoding an immunomodulator, preferably wherein said immunomodulator is a cytokine, and more preferably wherein said cytokine is preferably interleukin 6 or interferon-γ.

In still other preferred embodiments of the method, the heterologous DNA construct is inserted into the vaccinia MVA hemagglutinin locus, the vaccinia MVA thymidine kinase locus, or the fowlpox thymidine kinase locus, and preferably the foreign DNA further comprises a functional fowlpox thymidine kinase gene.

In accordance with still another aspect of the invention there is provided a recombinant eukaryotic cytoplasmic DNA virus producible by any of the above methods and/or preferred embodiments thereof. In a preferred embodiment, the recombinant eukaryotic cytoplasmic DNA virus is an attenuated poxvirus. In another preferred embodiment, the recombinant virus is mhaS, mha-gag-pol, mtkS, mtk-gagpol, mtk-1Ag, mtk-prME, mtk-e/IFN, mtk-eIL6, m-gpe-IFN, m-gpe-IL6, m-gpe, f-tks, ftk-prME, ftk-gagpol.

In accordance with still another aspect of the invention there is provided a vaccine comprising an attenuated recombinant eukaryotic cytoplasmic DNA virus prepared by the methods discussed above together with a pharmaceutically acceptable carrier. The vaccine is preferably a live vaccine.

In accordance with yet another aspect of the invention the use of a recombinant virus prepared by any one of the above methods for the vaccination of mammals is provided.

In accordance with yet another aspect of the invention there is provided a method of vaccination, comprising the step of administering a vaccine as described above to mammals, preferably a human patient. In preferred embodiments the vaccination is administered orally or parenterally.

In accordance with still another aspect of the invention there is provided a method of producing a vaccine, comprising the step of infecting cells with a recombinant eukaryotic cytoplasmic DNA virus prepared as described above.

In accordance with a further aspect of the invention there is provided a method of vaccinating an animal against a pathogenic virus comprising administering to the animal a vaccine prepared as described above.

Other objects, features and advantages of the present invention will become apparent from the following description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

### Brief Description of the Figures

FIGURE 1 outlines the transient marker stabilization technique, which requires the construction of a chimeric viral vector containing repeated DNA segments (S1), a color marker gene (cm), a selection marker gene (sm) and a foreign gene (fg).

FIGURE 2 outlines the construction of plasmid pZgpt-a.

FIGURE 3 outlines the construction of plasmids pφX-lo1/a and pφX-lo1/b.

FIGURE 4 outlines the construction of the plasmid pφX-gpt-lacZr.

FIGURE 5 outlines the construction of the plasmids pvA(1) and pvA(2).

FIGURE 6 outlines the construction of the plasmid pHA-Mega.

FIGURE 7 outlines the construction of the plasmid pHA-vA(2)a.

FIGURE 8 outlines construction of the modified virus mhaS. The angled arrows indicate the annealing position of the PCR primers.

FIGURE 9 outlines the construction of the plasmid pSep(1) and shows the sequence of the strong early/late promoter Sep.

FIGURE 10 outlines the construction of the plasmid pvA-gagpol.

FIGURE 11 shows a Western blot of total cellular proteins from cells infected with vaccinia MVA isolates or control viruses. Arrows indicate the p55 (*gag* precursor) and p41 bands.

FIGURE 12 outlines the construction of the plasmid pTZgpt-TK⁺lacZa.

FIGURE 13 outlines the construction of the plasmids ptkS-a and ptk-vA.

FIGURE 14 shows a Western blot analysis of cell lysates after infection of CEFs with MVA viruses expression *gagpol*. Lanes 1-8 are white plaque isolates #1-8, lane 9 is vaccinia vgagpolIIIB#9 as a positive control, lane 10 wild-type MVA as a negative control, lane 11 lysates of mock-infected cells, lane 12 HIV infected cells as a positive control, lane 13 mock infected cells. The arrows indicate the positions of the p55, p41, and p24 bands.

FIGURE 15 outlines the construction of the plasmid pvA(1)-preS1dMyr.

FIGURE 16 outlines the construction of the plasmid ppME273.

FIGURE 17 outlines the construction of the plasmid pvA-pME.

FIGURE 18 outlines the construction of the plasmids pTKm-vA(1) and pTKm-vA(2).

FIGURE 19 outlines the construction of the fowlpox virus f-tkS.

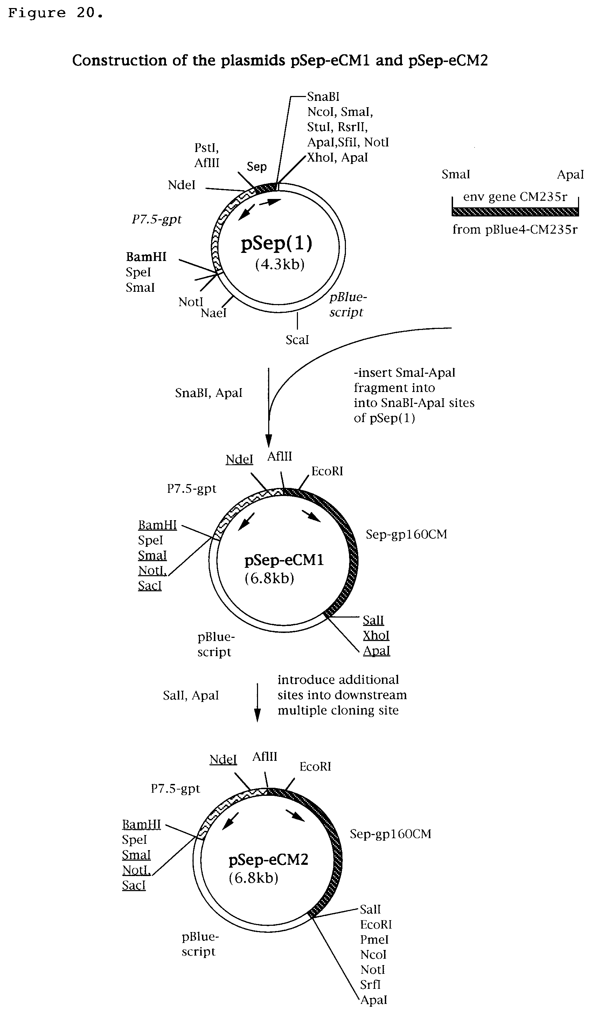

FIGURE 20 outlines the construction of the plasmid pSep-eCM2.

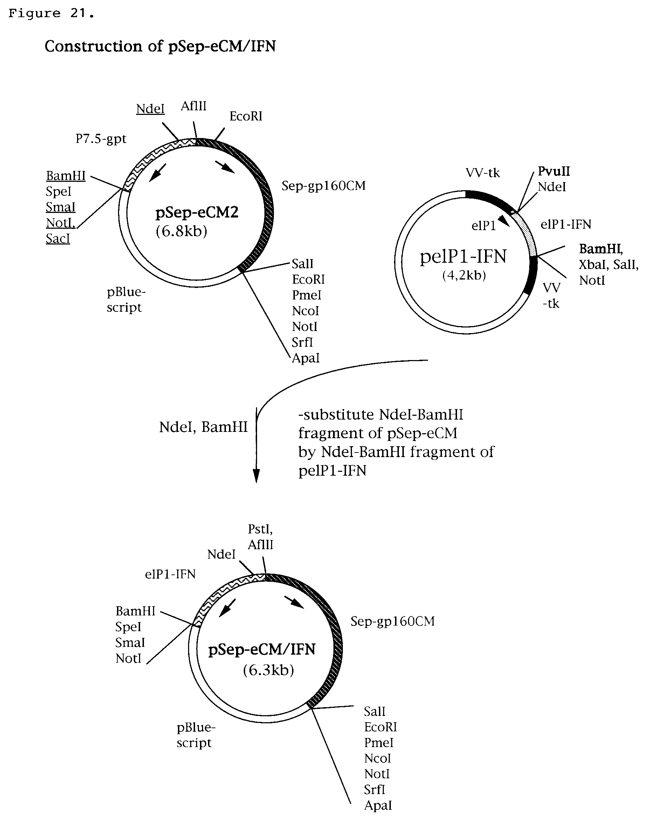

FIGURE 21 outlines the construction of the plasmid pSep-eCM/IFN.

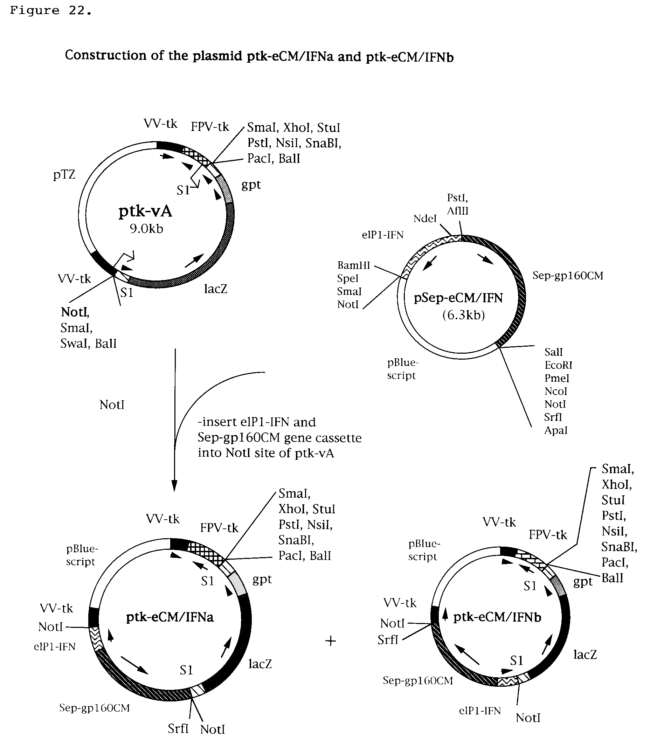

FIGURE 22 outlines the construction of the plasmids ptk-eCM/IFNa and ptk-eCM/IFNb.

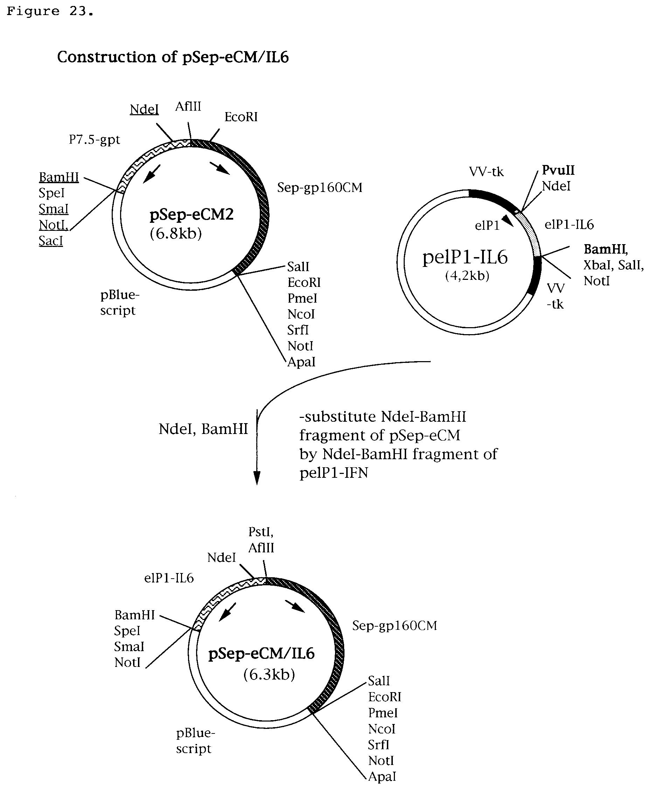

FIGURE 23 outlines the construction of the plasmid pSep-eCM/IL6.

FIGURE 24 outlines the construction of the plasmids ptk-eCM/IL6a and ptk-eCM/IL6b.

FIGURE 25 outlines the construction of the plasmid pSep2-CM235r2.

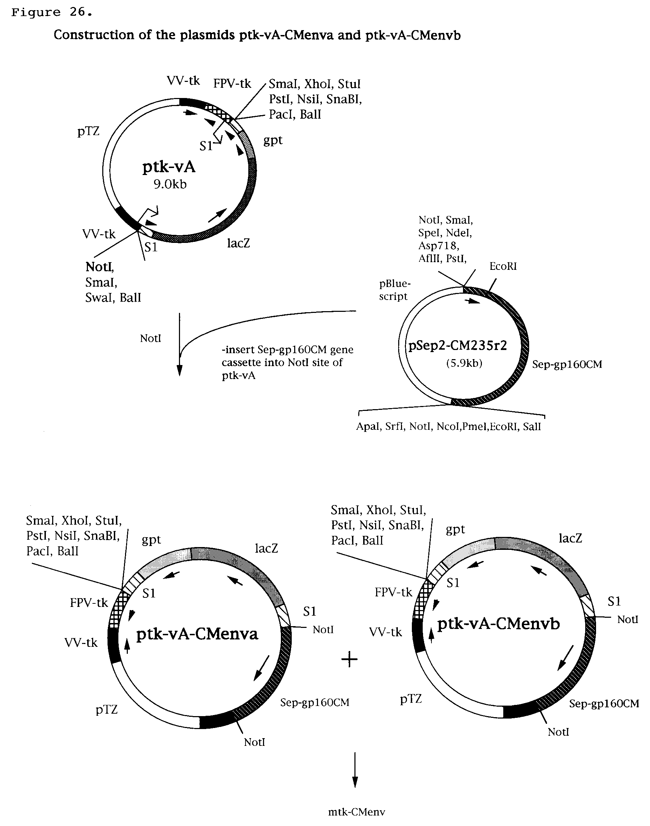

FIGURE 26 outlines the construction of the plasmids ptk-vA-CMenva and ptk-vA-CMenvb.

### Detailed Description of Preferred Embodiments

The present invention provides an efficient method for producing recombinant eukaryotic cytoplasmic DNA viruses that contain foreign DNA stably integrated into the viral genome, but that do not contain a selection marker gene. The method, designated as transient marker stabilization (TMS), can be used to efficiently produce recombinant viruses from highly attenuated strains of eukaryotic cytoplasmic DNA viruses, such as the vaccinia MVA strain described above. The method proceeds through initial construction of a virus that contains selection marker genes within the genome. These marker genes then are lost from the viral genome by genetic recombination during subsequent propagation of the virus, resulting in a stable recombinant virus. Recombinant eukaryotic cytoplasmic DNA viruses produced by the method can be used as live vaccines.

The invention is based on the introduction of a heterologous DNA construct into the genome of a eukaryotic cytoplasmic DNA virus *via* either direct molecular cloning (Scheiflinger et al.,supra,) or *in-vivo* homologous recombination. See, for example, Mackett *et al*., *supra*, and Panicali et al.,supra,. As used in the present context, "eukaryotic cytoplasmic DNA virus" includes poxviruses and iridoviruses. "Poxvirus" includes any member of the family *Poxviridae*, including the subfamilies *Chordopoxviridae* (vertebrate poxviruses) and *Entomopoxviridae* (insect poxviruses). See, for example, B. Moss in VIROLOGY , ed. Fields et al., Raven Press 2: 2079-2111 (1990). The chordopoxviruses comprise, *inter alia*, the following genera from which particular examples are discussed herein, as indicated in parentheses: *Orthopoxvirus* (vaccinia); *Avipoxvirus* (fowlpox); *Capripoxvirus*; *Leporipoxvirus*; and *Suipoxvirus*. The entomopoxviruses comprise three genera designated A, B and C. "Iridovirus" includes any virus that is classified as a member of the family *Iridoviridae*, as exemplified by the African swine fever virus as well as certain amphibian and insect viruses. Any eukaryotic cytoplasmic DNA virus can be used to practice this invention, but vaccinia virus and fowlpox virus are preferred. The TMS method, however, works also with DNA viruses in general including adenovirus, herpes- and baculovirus.

The heterologous DNA construct comprises a foreign DNA sequence that is to be stably retained by the virus, together with genes encoding at least one dominant selection marker and at least one color selection marker, both of which are ultimately lost from the viral genome. As used hereinafter the term "foreign DNA" applies only to DNA sequences that are intended to be stably retained by the virus. The foreign DNA can contain, for example, one or more genes encoding proteins that are intended for expression by the virus, or can include sequences, such as restriction endonuclease recognition sites, that are intended to facilitate further genetic manipulation of the virus. Other foreign DNA sequences may also be used that would be readily apparent to the skilled practitioner.

A dominant selection marker gene is herein defined as a gene whose protein product provides a growth advantage to a virus encoding the protein. Dominant selection markers include the E. coli genes xanthine guanine phosphoribosyltransferase (gpt), hygromycin phospotransferase (hph) and neomycin-phosphotransferase (neo).

In a preferred embodiment of the invention, the presence of a dominant selection marker gene permits survival of viruses under conditions that are lethal to viruses lacking the gene. In a further preferred embodiment, the presence of a dominant selection marker gene permits survival of viruses in the presence of a drug or combination of drugs that are lethal to viruses that lack the gene. The dominant selection marker gene is different from the foreign gene or the color selection marker gene.

A color selection marker gene is herein defined as a gene whose protein product allows visual selection of cells that are infected with a virus encoding the gene. In a preferred embodiment of the invention, cells containing the color selection marker gene can be distinguished by virtue of being a different color from cells that do not contain the gene. In a particularly preferred embodiment of the invention the protein product of the color selection marker gene is an enzyme that converts a substrate to a differently colored product. The color selection marker gene is different from the foreign gene or the dominant selection marker gene.

The dominant selection and color marker genes are placed within the construct so that when the construct is incorporated into a recombinant virus, the marker genes are bound by the pair of repeat DNA sequences. As used herein, the term "direct repeat" indicates one of a pair of DNA sequences that are sufficiently similar in sequence to allow genetic recombination to occur between the direct repeats during propagation of a recombinant virus, thereby eliminating one of the direct repeats from the viral genome together with any DNA sequence that was originally bound by the pair of repeats. In a preferred embodiment of the invention the direct repeats will have identical DNA sequences.

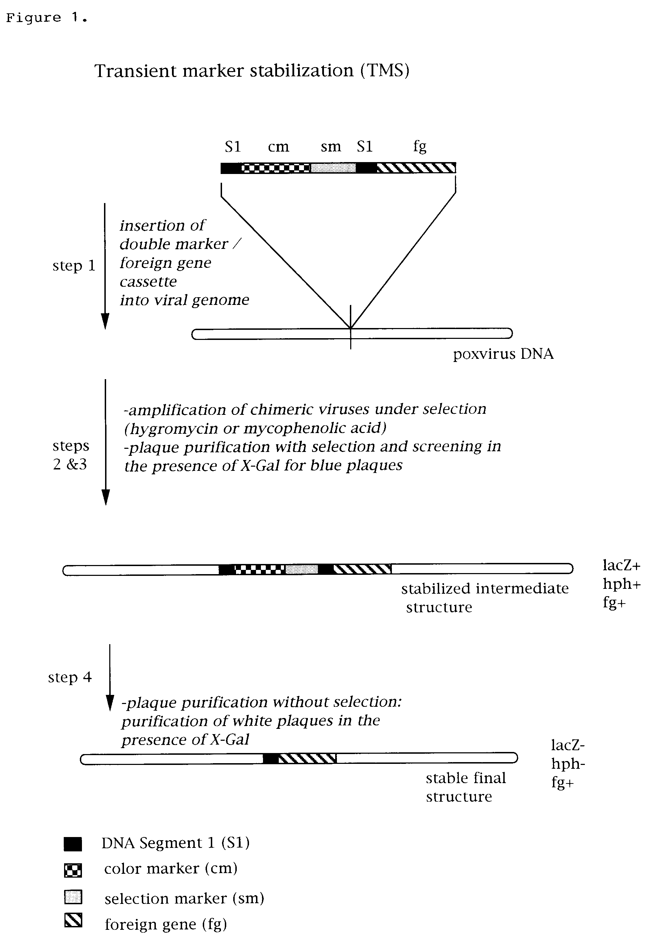

During initial propagation of the virus the marker genes are expressed, thereby allowing, by appropriate choice of growth conditions, efficient selection of cells containing recombinant viruses among a background of cells that predominantly contain wild-type virus. Once the growth conditions are changed to remove selective pressure genetic recombination can occur, and during subsequent plaque purification of the virus, the DNA sequence between the direct repeats is lost from the viral genome together with one of the repeats. This results in a pure recombinant virus containing stably integrated foreign DNA but lacking the selection marker genes. The transient marker stabilization method is illustrated schematically in Figure 1, in which "S1" denotes a direct repeat, "cm" denotes a color selection marker gene, "sm" is a dominant selection marker gene, and "fg" is foreign DNA.

The first step in the TMS method is to prepare a DNA construct that contains the desired foreign DNA, the selection markers, and the direct repeats in the correct relative configuration for insertion into the genome of a eukaryotic cytoplasmic DNA virus. In a preferred embodiment of the invention, the construct is a plasmid vector, thereby facilitating manipulation of the construct in bacterial cells, such as *E. coli*.

Suitable plasmid vectors may be constructed anew for each foreign DNA, but it is preferred to initially construct a vector containing the selection markers, the direct repeats, and at least one multiple cloning site (MCS). A variety of foreign DNA segments may then be readily inserted into the multiple cloning site by standard techniques of molecular biology without the need to construct a new vector each time. See Sambrook *et al*., MOLECULAR CLONING: A LABORATORY MANUAL, Cold Spring Harbour Laboratory Press, 1989).

The selectable marker genes, direct repeats, and multiple cloning sites are arranged within the vector such that the marker genes are placed between direct repeats, and an MCS is placed either 5' to the first (*i.e*., 5') direct repeat or 3' to the second (*i.e*., 3') direct repeat, or both. This placement ensures that when the vector is linearly integrated into a recombinant virus, the marker genes, but not the MCS, are bound by repeat DNA sequences. As used herein, the term "bound by" refers to the configuration obtained once the DNA construct is linearly inserted (that is, inserted as a linear molecule) into a eukaryotic cytoplasmic DNA virus genome.

DNA that is cloned into an MCS according to the present invention will be stably retained whilst the elements between the genetically unstable direct repeats are lost. Suitable arrangements of the genetic elements required for practising the TMS technique include, but are not limited to:
5'→MCS-direct repeat-promoter-color selection marker-promoter-dominant selection marker-direct repeat→3';
5'→direct repeat-promoter-color selection marker-promoter-dominant selection marker-direct repeat-MCS→3';
5'→MCS-direct repeat-promoter-dominant selection marker-promoter color selection marker-direct repeat→3';
5'→direct repeat-promoter-dominant selection marker-promoter-color selection marker-direct repeat-MCS→3'.

When the vector is a circular plasmid the 5' and 3' ends of the arrangements shown above will be covalently linked. Vectors with the structure described above can be constructed by standard molecular cloning techniques, as illustrated in Example 1. Genes encoding color markers and dominant selection markers are readily available from commercial suppliers (for example, Life Technologies, Gaithersburg, MD; Promega Corp., Madison, WI; Stratagene, San Diego, CA; Invitrogen, San Diego, CA). The genes are typically contained within plasmid vectors, and may be released from the vectors by digestion with one or more restriction endonucleases. Examples of suitable color selection markers are the *E. coli* genes *β-galactosidase* and glucuronidase. Examples of suitable dominant selection markers are the *E. coli* guanine phosphoribosyl transferase (*gpt*) gene (Falkner et al., supra) and hygromycin phosphotransferase (*hph*) (Zhou et al., *supra*). If the selection markers and direct repeats are bound by convenient restriction endonuclease sites they may simply be ligated into the vector at the desired sites by standard methods

Alternatively, restriction endonuclease sites may be engineered onto the 5' and 3' termini of the genes by standard techniques such as, for example, oligonucleotide ligation or the polymerase chain reaction (PCR). A variety of other methods of achieving the desired arrangement of the direct repeat and marker sequences will be apparent to the skilled practitioner. See Sambrook, *et al*., *supra*, and Ausubel *et al*., *supra*. The marker genes are placed under the control of a suitable promoter by standard techniques. See Sambrook *et al*., *supra*. Suitable promoters include poxvirus early/late promoters such as the vaccinia P7.5 promoter (Cochran, M. A., Puckett, C. and Moss, B. J. Virol. 54: 30-37, 1985), the thymidine kinase promoter (Weir, J. P. and Moss, B. Journal of Virology 46: 530-537, 1983) and the synthetic promoter elP1 (see Example 1). The color- and dominant-selection marker genes may be under the control of the same or different promoters. In a preferred embodiment each gene is under the control of a copy of the weak early/late promoter elP1.

If the vector is to be introduced into a viral genome by homologous recombination, it must also contain flanking regions that are homologous to the desired insertion locus within that genome. For a general description of the use of homologous recombination to construct recombinant vaccinia viruses, see Mackett, *et al*., "The Construction and Characterization of Vaccinia Virus Recombinants Expressing Foreign Genes" in DNA CLONING: A PRACTICAL APPROACH, D.M. Glover, Ed., IRL Press, Oxford, 1985. The insertion locus must either be within a gene which is not essential for viral growth in cells, or be within an intergenic section of the genome which similarly does not interfere with viral growth. Attenuated eukaryotic cytoplasmic DNA viruses often have lost portions of their genome after multiple passages in culture, and it is frequently necessary, therefore, to ensure that an intended insertion locus is still present within the viral genome prior to attempting construction of a recombinant virus. This can be achieved by standard techniques such as Southern blotting using a probe homologous to the desired insertion locus, or by the PCR using primers that anneal within, or close to, the insertion site. Characterization of the hemagglutinin locus of the attenuated vaccinia virus MVA in this fashion is demonstrated in Example 2. In a preferred embodiment of the invention, the desired insertion point within a vaccinia virus is within either the hemagglutinin (HA) locus or the *tk* locus. In another preferred embodiment of the invention the desired insertion point within a fowlpox virus is within the *tk* locus. It is particularly preferred that, when a foreign DNA is inserted within the *tk* locus of a poxvirus, a functional copy of a poxvirus *tk* gene is included within the foreign DNA. See *infra*. Other suitable insertion points will be apparent to the skilled practitioner.

Once a suitable insertion locus has been determined, nucleotide sequences surrounding the desired insertion site are incorporated as flanking regions in the TMS vector described above. This can be achieved by standard techniques developed for homologous *in vivo* recombination. See Mackett *et al*., *supra*, and references therein.

In a preferred embodiment of the invention the TMS vector is introduced into the virus by the direct molecular cloning method (Scheiflinger et al., supra). In this technique the linearized TMS vector is ligated into a unique restriction site within the desired insertion locus in the viral genome. In the context of this invention, a unique restriction site is one that does not occur anywhere else within the viral genome. A unique site may fortuitously occur within an insertion locus, or more typically the virus genome is engineered to introduce the site. The site is preferably introduced by homologous recombination using the TMS method.

To accomplish this engineering, a vector is constructed essentially as described above, containing sequences homologous to the insertion locus that flank two direct repeat sequences. The direct repeats in turn flank at least one color marker gene and at least one dominant selection marker gene. Instead of carrying a gene encoding a desired protein, however, the vector carries a DNA segment bearing one or more unique restriction sites. This segment can be either 5' to the first (*i.e.* 5') direct repeat or 3' to the second (*i.e.* 3') direct repeat. A second DNA segment may also carry the same, or different, unique restriction sites at the end of the construct opposite from the first segment (see Example 3).

When the vector is introduced into the virus, and the virus is subsequently propagated (see below), the selection markers between the direct repeats ultimately are lost, whilst the unique restriction site(s), being outside the direct repeats, stably integrates into the viral genome. Example 3 provides an illustration of the introduction of two *Srf*I sites into the hemagglutinin locus vaccinia MVA virus genome and Example 8 illustrates the introduction of a *Sma*I site within the *tk* locus of the MVA virus.

Once a suitable vector for carrying the gene encoding the desired protein is constructed, the gene encoding the desired foreign protein is inserted into the vector by standard methods. See Sambrook *et al., supra*. Although it is possible to construct a vector to contain a suitable promoter controlling expression of the desired protein, the DNA construct inserted into the vector typically contains the desired protein gene already under the control of a suitable promoter. A suitable promoter is the semi-synthetic early/late promoter Sep (EP 0 561 034). The vector may be used to introduce more than one foreign gene into the viral genome. For example, instead of ligating a single gene construct into the MCS of the vector, a construct containing two genes may be used, or two constructs may sequentially be ligated into the vector. Each gene is preferably under the control of its own promoter.

In a preferred embodiment of the invention the second gene encodes an immunomodulator, such as an interleukin or an interferon. When a recombinant virus containing genes for a first protein and a gene for an immunomodulator is used to infect an animal host, the presence of the immunomodulator causes a potentiation of the host's immune response against the first protein.

The protein products can be expressed as distinct proteins or as fused proteins. The construction and use of vectors encoding two genes is illustrated in Examples 15-19.

The vector is then introduced into the host virus either by homologous recombination, or by direct molecular cloning. If the direct molecular cloning method is used, isolated viral DNA is first digested with a restriction endonuclease that cleaves the viral DNA at a unique site within the desired insertion locus. The vector is then digested with the same endonuclease, and the desired fragment is ligated into the viral DNA. The viral DNA is then transfected into cells infected with wild-type virus and the virus is allowed to replicate for several days without any dominant selection or color selection. The transfection is preferably carried out by the calcium phosphate method. See Graham *et al., Virol*. 52: 456-467 (1973).

Crude viral stocks are then prepared from the cells and are used to infect host cells under dominant selection conditions. After several days the color selection agent is added, and a few hours later colored viral plaques are picked for further purification. Two additional rounds under dominant selection and color screening are performed. Three rounds of purification are then carried out without dominant selection in order to allow elimination of the genetically unstable DNA segments located between the direct repeat sequences. At the end of the third round of purification the color selection agent is added, and white plaques are purified a further two times in the presence of the color selection agent, each time selecting white plaques. Typically, at the end of the last round of purification all the viral plaques are white, indicating that a homogeneous recombinant virus lacking selectable markers has been prepared. This can be confirmed by analysis of the viral genome by Southern blot, using a probe that recognizes the gene encoding the desired foreign protein, or by the PCR. In addition, expression of the desired protein can be assayed by Western blot using an antibody to the protein.

If the homologous *in vivo* recombination method is chosen to introduce the heterologous DNA construct into the virus, the plasmid is transfected into cells previously infected with wild-type virus. The various rounds of plaque purification with and without selection are then carried out as described above until a homogeneous recombinant virus, lacking selectable markers, is obtained. The resultant virus is then assayed for the presence of foreign DNA as described above.

As described *supra*, the TMS method is particularly suited to construction of recombinant viruses from attenuated strains of poxvirus that are preferred for use in vaccines. Prior to the present invention there has been a long-felt need for a recombinant poxvirus that is useful for vaccine purposes, but that is not unduly virulent. The level of viral virulence is particularly important when live viral vaccines are to be used in immunocompromised patients. Heretofore, recombinant poxviruses based on a strain with an established safety record have not been available.

As shown in the examples below, recombinant MVA vaccinia viruses can be reproducibly obtained by the TMS method by inserting foreign genes into the native MVA *tk* locus together with a functional fowlpox *tk* gene (See Example 8 *et seq.*). Previous methods of making recombinant MVA, such as those described by Altenburger *et al.,* US Patent 5,185,146, produce a virus totally lacking *tk* activity. As discussed above, this can render the recombinant virus nonviable even though, in the wild type virus, the *tk* gene is nonessential.

The vaccinia MVA strain employed herein was passed more than 570 times in chicken embryo fibroblasts. Stickl *et al., supra,*. The virus lost some of its host range functions and six major deletions totalling 31kb occurred in its genome. Mayr *et al., Infection* 3: 6-14 (1975); Altenburger *et al., Arch. Virol.* 105: 15-27 (1989); Meyer *et al., J. Gen. Virol.* 72: 1031-38 (1991). The MVA virus is considered "highly attenuated" because it is severely restricted in its host range and cannot grow in human and several other types of mammalian cells.

The viruses prepared by the TMS method are useful in the preparation of vaccines. These vaccines are amenable to the conventional routes and modes of administration. One preferred route of vaccination using the MVA strain of vaccinia is oral application. This route of vaccination has proven to induce specific immunoglobulins of the A class (IgA) that are important for mucosal immunity. To enhance this type of immunity, a copy of a gene encoding an immunomodulator such as human interleukin-6 (IL-6) can be inserted into an MVA isolate. Chicken embryo fibroblasts suitable for propagating attenuated poxviruses can be prepared according to the methods described in the European Patent application EP 0338 807 A2. Experiments with the MVA strain have been reviewed recently (Mahnel and Mayr, Berl. Münch. Tierärztl. Wochenschr. 107 (8) p.253-256, 1994). In all examples described below, MVA virus was grown in CEF cells and purified according to Joklik, Virol. 18: 9-18,1962. Vero cells (ATCC# CCL 81) were obtained from the American Type Culture Collection.

### Example 1. Construction of plasmid vectors for use in the Transient Marker Stabilization technique.

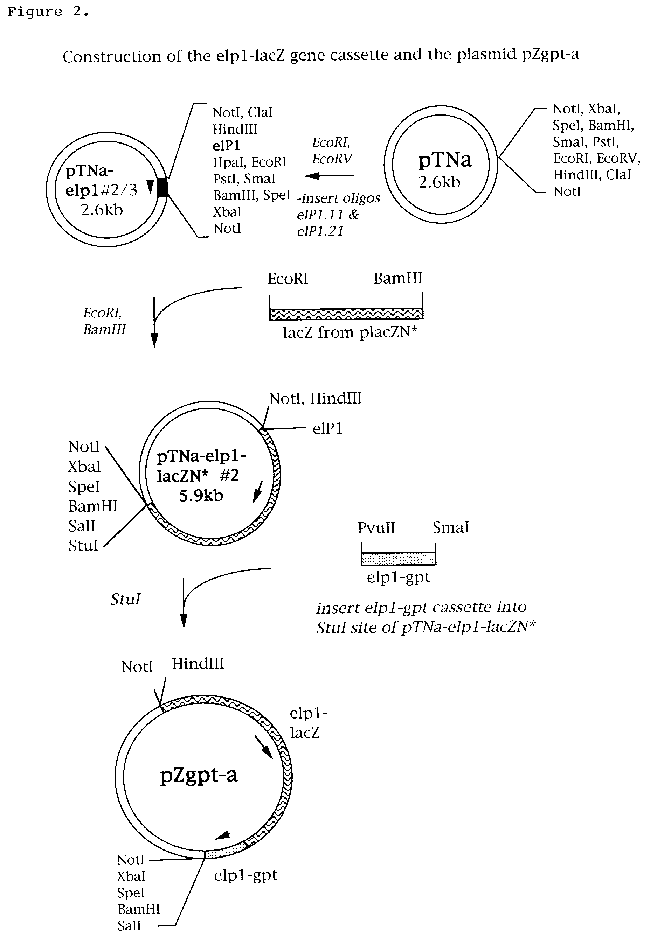

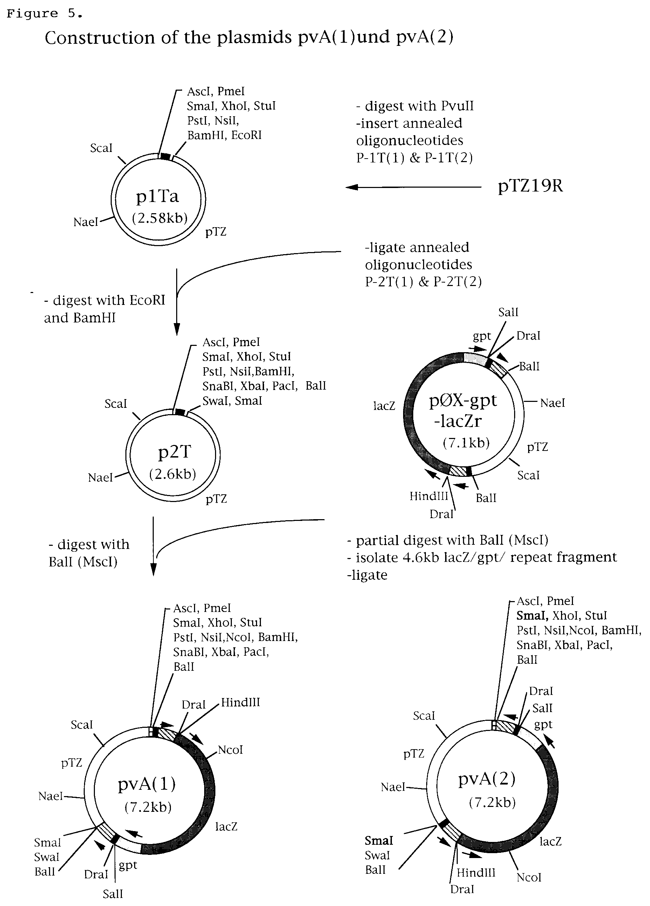

Plasmid vectors suitable for the transient marker stabilization technique are shown in Figure 5. These vectors contain multiple cloning sites for insertion of foreign genes. The vectors are designated pvA(1) and pvA(2) and each is composed of a double marker cassette consisting of either the *gpt* or *hph* gene as the dominant selection marker and the *lacZ* gene as the color marker, flanked by repeated DNA segments. These plasmids were constructed according to the schemes shown in Figures 2-5 as described in detail below.

### Construction of pZgpt-a

The first step in vector construction was to place an appropriate lacZ gene cassette under the control of a weak early/late promoter. Thus, the multiple cloning site of the plasmid pN2 (see Example 1 of EP 0 561 034) was inserted as a NotI fragment into the plasmid pTZ-N (see Example 5 of EP 0 561 034) to produce pTNa. The annealed oligonucleotides elpl.11 (5'-GGG TAA AAG TTG AAA ATA TAT TTC TAT GCT ATA AAT AAG TTA ACG-3')(SEQ ID NO:1) and elp1.21 (5'-AAT TCG TTA ACT TAT TTA TAG CAT AGA AAT ATA TTT TCA ACT TTT ACC C-3') (SEQ ID NO:2) were then inserted between the EcoRI and EcoRV restriction sites of pTNa to produce plasmid pTNa-elp1#2/3. The lacZ fragment from plasmid placZN*, (plac ZN* was constructed by digesting the plasmid pFP-Zsart (EP 0 538 496) with *Not*I and ligating pFP-Zsart with the oligonucleotide P-*Not*I⁻ (5'-GGCCAT-3') was then inserted as an EcoRI-BamHI fragment between the EcoRI and BamHI sites of plasmid pTNa-elp1#2/3, resulting in the plasmid pTNa-elp1-lacZN*#2, which contains the desired gene cassette, flanked by several restriction sites (Figure 2).

The next step was to construct the double marker cassette consisting of the *lacZ* and the *gpt* genes. For this purpose the plasmid pelp1-gpt was constructed. First, the gpt-gene was amplified with the primers ogpt-f (5'ATC ATA TGA GGA TCC ATG AGC GAA AAA TAC ATC GTC ACC-3') (SEQ ID NO:3) and ogpt-r (5'-ATC CCG GGA CGT CAT AAA AAG ATT AGC GAC CGG AGA TTG GCG G-3') (SEQ ID NO:4) using as template the plasmid pSV2-gpt (Mulligan, R. and Berg, P. Proc. Natl. Acad. Sci. USA 78, 2072-2076, 1981). The PCR product was cleaved with NdeI and SmaI and inserted into the corresponing sites of the plasmid pFS51 (EP 0 538 496) resulting in the plasmid pdP-gpt. The elp1 promoter, in the form of the annealed oligonucleotides oelp1.1 (5'- TAT GTA AAA GTT GAA AAT ATA TTT CTA TGC TAT AAA TAA GTT AAC A -3') (SEQ ID NO:5) and oelp1.2 (5'-GAT CTG TTA ACT TAT TTA TAG CAT AGA AAT ATA TTT TCA ACT TTT ACA-3') (SEQ ID NO: 6) was finally inserted between the NdeI and BamHI sites of pdP-gpt resulting in the plasmid pelp1-gpt. The *elp1-gpt* gene cassette was removed as a *Pvu*II-*Sma*I fragment from the plasmid pelp1-gpt and inserted into the unique *Stu*I site of pTNa-elp1-lacZN*#2. Plasmid pTNa-elp1-lacZN*#2 was cut with *Stu*I, treated with calf intestine phosphatase and ligated with the elp1-gpt gene cassette to produce plasmid pZgpt-a. The double marker can be excised from the resulting plasmid pZgpt-a at several restriction sites including *Not*I (Figure 2).

### Construction of pφX-lo1/a

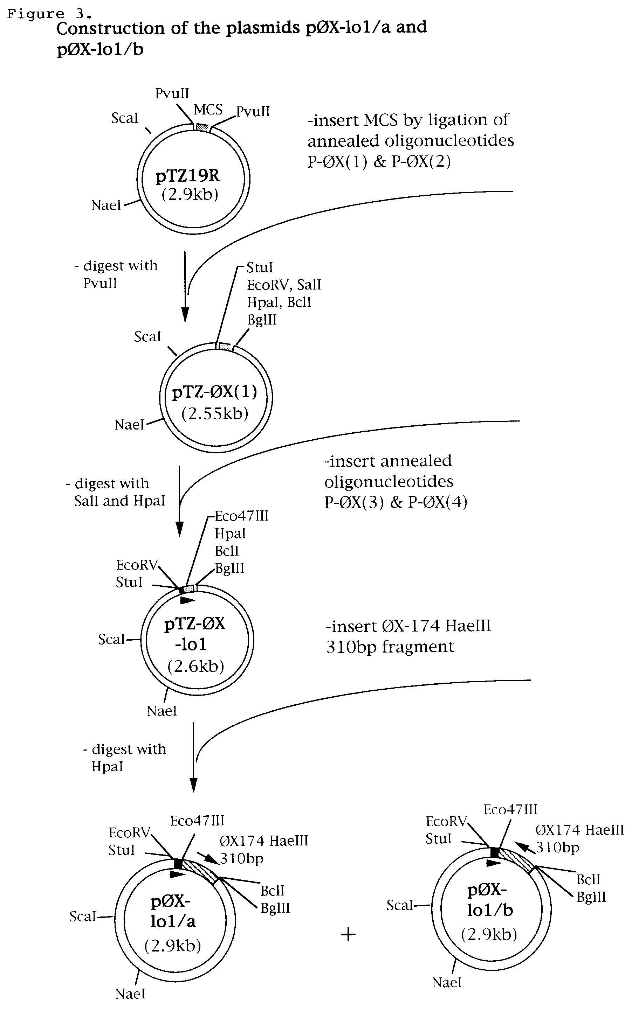

The next step in the generation of the vectors of the pvA-series was the construction of the plasmid pφX-lo1/a (Figure 3). This plasmid contains one of the DNA repeats intended to flank the double marker gene cassette (designated S1 in Figure 1). The repeat is flanked by suitable restriction sites needed for the further cloning steps.

Thus, the annealed oligonucleotides P-φX(1), (5'-AGGCCTGATA TCGTCGACGT TAACTGATCA AGATCT-3') (SEQ ID NO:7), and P-φX(2), (5'-AGATCTTGAT CAGTTAACGT CGACGATATC AGGCCT-3') (SEQ ID NO:8), were ligated with the large PvuII vector fragment obtained after cleavage of pTZ19R (Pharmacia, Inc., Piscataway, NJ) producing pTZ-φX(1). This plasmid was cut with *Sal*I and *Hpa*I and ligated with the oligonucleotides P-φX(3), (5'-TCGAAATAAC TTCGTATAAT GTATGCTATA CGAAGTTATA GCGCTGTT-3') (SEQ ID NO:9), and P-φX(4), (5'-AACAGCGCTA TAACTTCGTA TAGCATACAT TATACGAAGT TATT-3') (SEQ ID NO:10), to produce plasmid pTZ-φX-lo1.

The plasmid pTZ-φX-lo1 was digested with *Hpa*I and ligated with the 310bp *Hae*III DNA fragment prepared from the phage φX-174 (phage DNA was obtained from New England Biolabs, Inc., Beverley, MA) to produce pφX-lo1/a and pφX-lo1/b.

### Construction of pvA(1) and pvA(2)

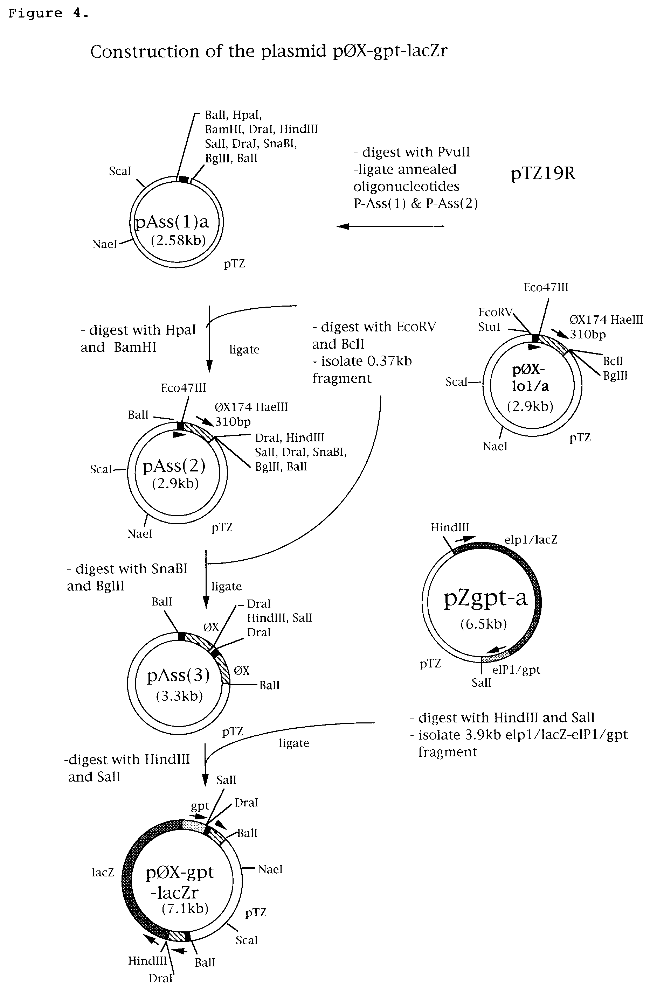

A further step in the generation of the pvA-series plasmids was the construction of the plasmid pφX-gpt-lacZr (Figure 4) from which the double marker gene cassette flanked by the repeats S1 was excised as a blunt ended restriction fragment. The double marker cassette was inserted into the intermediate plasmid p2T, resulting in the final plasmids pvA(1) and pvA(2) (Figure 5).

Thus, the annealed oligonucleotides P-Ass(1), (5'-GTGGCCAGTT AACGGATCCT TTAAAAAGCT TGTCGACTTT AAATACGTAA GATCTTGGCC AA-3') (SEQ ID NO:11), and P-Ass(2), (5'-TTGGCCAAGA TCTTACGTAT TTAAAGTCGA CAAGCTTTTT AAAGGATCCG TTAACTGGCC AC-3') (SEQ ID NO:12), were ligated with the large vector fragment obtained after *Pvu*II digestion of pTZ19R (Pharmacia, Inc.) to produce pAss(1)a (Figure 4).

pAss(1)a was cut with *Hpa*I and *Bam*HI and ligated with the 0.37kb fragment obtained by digestion of the plasmid pφX-lo1/a with *Eco*RV and *Bcl*I resulting in pAss(2). pAss(2) was in turn cut with *Sna*BI and *Bgl*II and again ligated with the 0.37kb fragment obtained after digestion of plasmid pφX-lo1/a with *Eco*RV and *Bcl*I resulting in pAss(3). pAss(3) was cut with *Hind*III and *Sal*I and ligated with the double marker cassette obtained as a *Hind*III-*Sal*I fragment from the plasmid pZgpt-a to produce pφX-gpt-lacZr.

The annealed oligonucleotides P-1T(1), (5'-AGTTTAAACG GCGCGCCCGG GCTCGAGAGG CCTCTGCAGA TGCATCCATG GGGATCCGAA TTC-3') (SEQ ID NO:13), and P-1T(2), (5'-GAATTCGGAT CCCCATGGAT GCATCTGCAG AGGCCTCTCG AGCCCGGGCG CGCCGTTTAA ACT-3') (SEQ ID NO:14), were ligated with the large vector fragment obtained after *Pvu*II digestion of pTZ19R (Pharmacia, Inc.) resulting in p1Ta (Figure 5).

p1Ta was digested with *Eco*RI and *Bam*HI, and ligated together with the annealed oligonucleotides P-2T(1), (5'-GATCCTACGT ATCTAGAATT AATTAATGGC CAATTTAAAT GCCCGGGA-3') (SEQ ID NO:15), and P-2T(2), (5'-AATTTCCCGG GCATTTAAAT TGGCCATTAA TTAATTCTAG ATACGTAG-3') (SEQ ID NO:16), to produce p2T.

Plasmid p2T was linearized with *Bal*I (or the isoschizomer *Msc*I) and ligated with the 4.6kb fragment released from the plasmid pφX-gpt-lacZr after digestion with *Bal*I. This fragment contains the desired double marker cassette, including flanking segments. The final plasmids are orientational isomers and were designated pvA(1) and pvA(2) (Figure 5).

### Example 2. The hemagglutinin (HA) gene locus as insertion site for MVA based chimeras: characterization of MVA recombinants with insertions in the HA-locus.

The hemagglutinin (HA)-locus of vaccinia virus is a site into which foreign genes have successfully been integrated *e.g.* in the vaccinia virus WR strain (Flexner *et al., Vaccines 87: Modern Approaches to New Vaccines including Prevention of AIDS*, Brown *et al.* (Eds.), Cold Spring Harbour Laboratory Press, Cold Spring Harbour, NY (1987) pp. 380-383). The HA-gene is not essential for growth of the virus in mammalian cell culture, but some degree of viral attenuation is associated with the HA-negative phenotype in animals (Buller & Palumbo, 1992; In: Recombinant poxviruses pp. 235-267; eds. Binns, M. M. & Smith, G. L. CRC Press.

Insertions of foreign genes into the HA-locus of the vaccinia MVA strain have not thus far been described. The MVA strain lacks heme agglutinating functions (Meyer *et al., supra*) and, prior to the present invention, it was therefore unclear whether this locus is present and suited for insertions. Since MVA is highly attenuated and has lost most of its capacity to grow in mammalian cell lines the behaviour of MVA with inserts in the HA-region was not predictable and was of considerable interest.

To identify suitable insertion sites and to investigate the behaviour and the growth characteristics of MVA viruses with insertions in the HA-locus the E. coli *lacZ* and *gpt* genes were inserted into this locus by standard recombination techniques (Mackett, M., Smith,G.L. and Moss, B. 1985. In D. M. Glover (ed.), DNA cloning: A practical approach, p. 191-211, IRL Press, Oxford.) using plasmid pHAgpt-lacZb. To construct this plasmid, first the plasmid pHA was generated by inserting, between the two PvuII sites of pTZ19R (Pharmacia, Inc.), the 1.5kb HincII fragment spanning the vaccinia WR hemeagglutinin gene. By inserting the 1.7kb HpaI-DraI fragment out of plasmid pTKgpt-F1s (Falkner, F. G. and Moss, B. J. Virol. 62, 1849-1854, 1988) into the unique NruI site of pHA the orientational isomeric plasmids pHAgpt-oFa and pHAgpt-oFb were obtained. Insertion of the lacZ gene, obtained as a BamHI fragment out of plasmid pSC11 (Chakrabarti et al., Mol. Cell. Biol. 5: 3403-3409, 1985),into pHAgpt-oFb resulted in the plasmid pHAgpt-lacZb. Several *lacZ* and *gpt* positive viruses were selected and plaque purified four times. Identification and purification of plaques could be normally performed. Small virus stocks were prepared and used for infections of chicken embryo fibroblasts (CEF). CEFs were infected with 1 plaque forming unit (pfu) of the small virus stocks. After 72h total DNA was prepared, digested with *Hind*III or *Nco*I, separated on a 1% Agarose gel and further processed according to a standard Southern blot procedure (see Sambrook *et al.,* MOLECULAR CLONING: A LABORATORY MANUAL, Cold Spring Harbour Laboratory Press, 1989). The immobilized fragments were hybridized to a vaccinia HA-gene probe prepared from plasmid pHA. This plasmid was prepared by inserting the 1.5kb *Hinc*II fragment of the vaccinia strain WR containing the HA gene into the *Pvu*II sites of pTZ19R (Pharmacia, Inc.).

The results of the Southern blot showed that pure stocks of recombinant virus were obtained after four rounds of plaque purification. Thus, the expected band of about 3.4kb was found in the wild-type virus control (lane MVA WT) and a large fragment of about 9.0kb corresponding to the expected size for *lacZ/gpt* insertion into the HA locus in the recombinant viruses. No wild-type virus specific bands could be detected in the *Nco*I digested samples. In the *Hind*III digests the expected fragments of about 42 and 5.0kb were detected.

This analysis showed for the first time that the HA-locus is a suitable insertion site in the MVA strain. Although the HA locus had previously been shown to be a potential integration site in other vaccinia strains, the special properties of MVA such as extreme attenuation and highly restricted host range, could have resulted in impaired growth as shown for tk-negative viruses. The findings of this example were the basis for further experiments with the direct cloning MVA strain mhaS (see Example 3).

### Example 3. Construction of an MVA-based host virus strain with unique cloning sites in the hemagglutinin gene, mhaS, using transient marker stabilization.

As shown in Example 2, the hemagglutinin locus is a suitable insertion site into the MVA wild-type strain. This finding was the basis to construct the modified MVA strain mhaS that has several unique cloning sites located in the hemagglutinin gene, including *Sma*I, *Srf*I and I-*Sce*I. These sites were found not to be present in the MVA wild-type strain by digesting genomic MVA DNA with the respective enzymes (data not shown). The virus strain mhaS is desirable because the unique insertion sites are required for the construction of chimeras by direct molecular cloning.

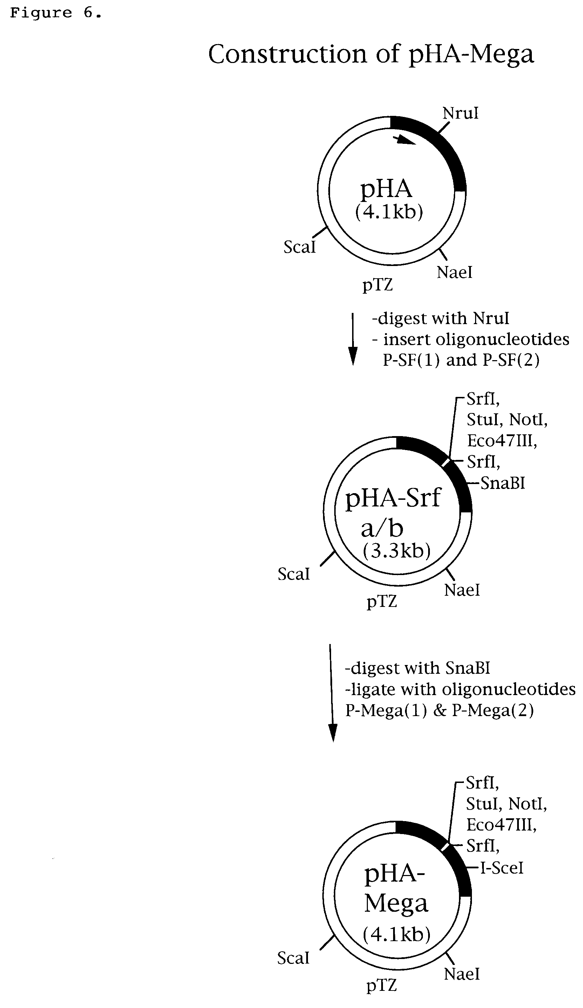

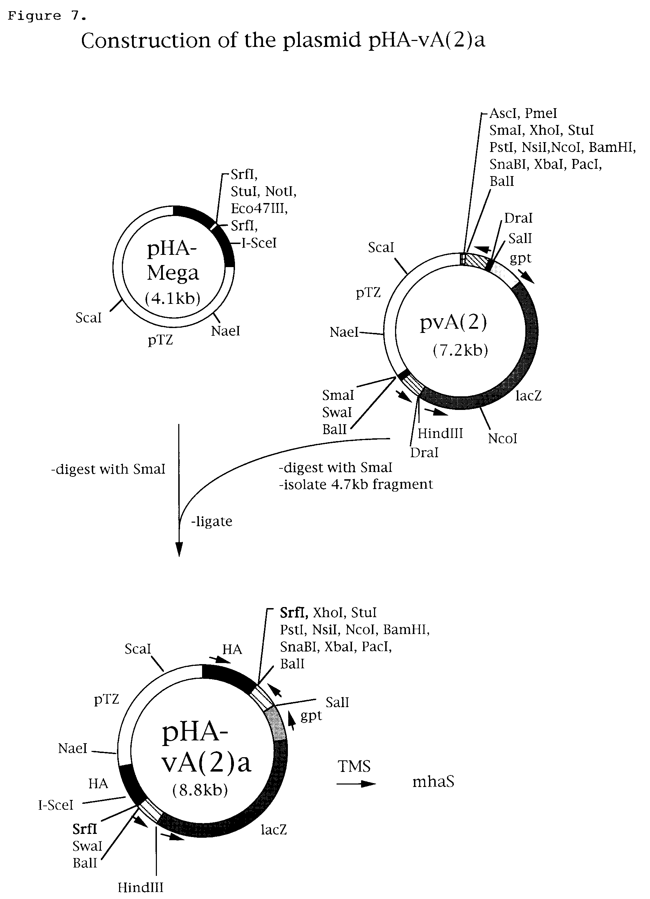

Prior to constructing the virus mhaS, the plasmids pHA-Mega and pHA-vA(2)a were constructed (Figures 6 and 7). The plasmid pHA-Mega has a multiple cloning site inserted into the former central *Nru*I site, containing several restriction sites, including an *Srf*I site, that does not occur in the vaccinia MVA genome. The site of the eight base cutter *Srf*I (GCCCGGGC) includes a *Sma*I site (CCCGGG). Additionally, an I-*Sce*I site, that also does not occur in the vaccinia MVA genome, was inserted adjacent to the multiple cloning site.

As shown in Figure 6, pHA-Mega was constructed by first digesting pHA with *Nru*I within the HA gene and inserting the annealed oligonucleotides P-SF(1), (5'-GCCCGGGCAG CGCTGCGGCC GCAGGCCTGC CCGGG-3')(SEQ ID NO:17), and P-SF(2), (5'-GCCCGGGCAG GCCTGCGGCC GCAGCGCTGC CCGGGC-3')(SEQ ID NO:18). The resultant plasmids were designated pHA-Srfa and pHA-Srfb. The unique *Sna*BI site within the HA gene of pHA-Srfa was used to insert the annealed oligonucleotides P-Mega(1), (5'-GGTAGGGATA ACAGGGTAAT AA-3')(SEQ ID NO:19), and P-Mega(2), (5'-TTATTACCCT GTTATCCCTA CC-3')(SEQ ID NO:20), resulting in the plasmid pHA-Mega.

The plasmid pHA-Mega was then digested with *Sma*I and ligated with the 4.7 kb *Sma*I fragment of pvA(2). The resulting plasmid was designated pHA-vA(2)a. In the plasmid pHA-vA(2)a, the double marker cassette including the DNA segments is flanked by vaccinia HA gene sequences.

The plasmid pHA-vA(2)a was used in a transient marker stabilization experiment as follows: 20 µg of the plasmid DNA of pHA-vA(2)a were precipitated according to the calcium-phosphate technique (Graham *et al., Virology* 52: 456-67 (1973)) and overlaid over CV-1 cells that were infected with 0.5 pfu of MVA wild-type virus one hour prior to the overlay. After 5-6 days of growth, a crude stock was prepared, followed by three plaque purifications under marker stabilization and three purifications without marker stabilization, during which the end product was formed.

Plaque assays were performed in chicken embryo fibroblasts under gpt-selection (transient marker stabilization) with X-Gal in the overlays. Under these conditions, gpt-positive viruses forming blue plaques can be purified. Plaque purification under stabilization was performed by plating 1/10, 1/100 and 1/1000 dilutions of the crude stock onto CEFs (10 petri dishes of each dilution) with an overlay including 1% low melting agarose and the ingredients for gpt-selection (Falkner & Moss , *J. Virol. 62*:1849 (1988)) with the modification that the concentration of mycophenolic acid was reduced to 1µg/ml in the overlay. After 3-4 days, the dishes were overlaid with a second agarose layer containing X-Gal (according to Chakrabarti *et al.,supra)*. After 12 hours (maximally 12-16h) blue plaques were picked and plated for the next round of purification.

The viruses were then subjected to three rounds of purification without selection in the presence of blue color screening. CEFs were infected with the virus suspension of the last (third) round and overlayed with medium containing 1% agarose without gpt selection. After 3-4 days, the dishes were overlaid with a second agarose layer containing X-Gal together with neutral red to visualize the plaques. In the first round without stabilizing gpt-selection about 30% of the plaques were white. White plaques were further purified two times resulting in 100% white plaques in the last round.

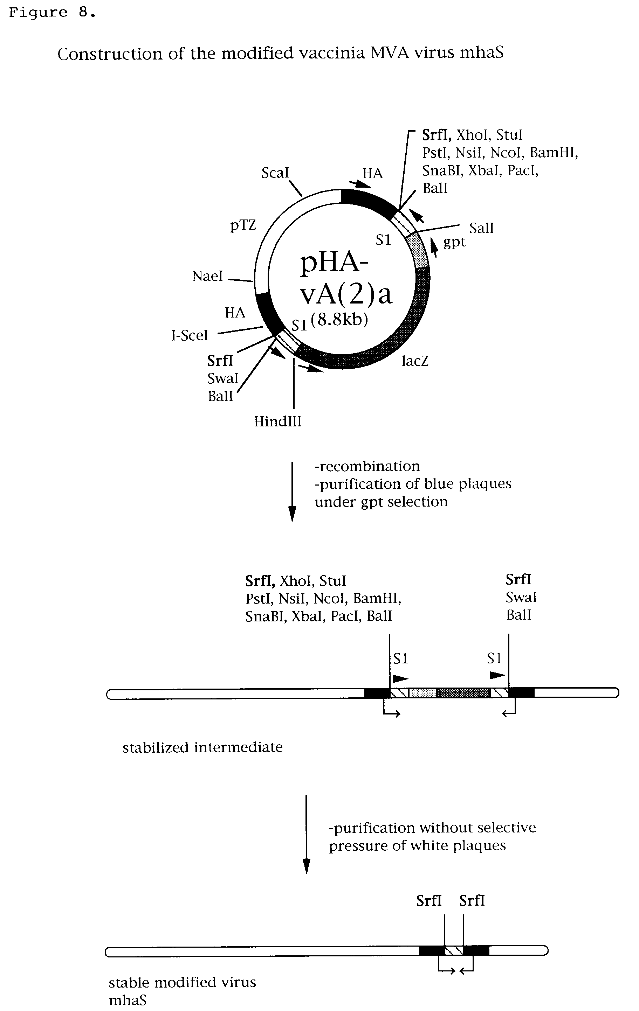

The structure of the finally purified virus in the white plaques is shown schematically in Figure 8. Recombination events due to the presence of the repeated elements S1 lead to loss of the marker genes when the selective pressure is removed. The final virus retains one of the S1 elements including the flanking restriction sites.

The structure of the viruses was confirmed by PCR analysis. For the PCR analysis the primers P-HA(1) (5'-GGA TCT ACA CAT TCA CCA GAA ACT A-3') (SEQ ID NO:21) and P-HA(2) (5'-CCG GAG TCT CGT CTG TTG TGG ATT CTC CA-3') (SEQ ID NO:22) were used to amplify the modified region in the HA-locus. Viruses with the correct insert gave rise to a PCR product of 0.6 kb, reflecting the insertion of one of the repeated elements, whereas the wild-type virus gave rise to a PCR product of 0.3 kb. Eleven plaques were screened for presence of the modification, and all were found to have the correct structure. In the negative control, as expected, no signal was seen while in the wild-type control the 0.3kb fragment appeared.

Three isolates were grown to large scale and purified over two sucrose steps. The final characterization concerning the modified region was done by cleaving the PCR products around the modified site with several enzymes (such as *Srf*I, I-*Sce*I and *Not*I). One of the isolates with the correct structure and normal growth was designated mhaS. This virus was used to insert gene cassettes by the direct molecular cloning technique.

### Example 4. Construction of chimeric MVA vaccinia viruses expressing the HIV-1 gagpol gene by transient marker stabilization.

The HIV proteins are preferred candidates for vaccine preparations either in subunit or in live vaccines. In this example the expression of the gagpol open reading frame in the MVA strain mhaS is described. The HIV gagpol gene is inserted into the hemagglutinin locus of the virus mhaS (see Example 3) and may be used as a live vaccine to prime human immune response.

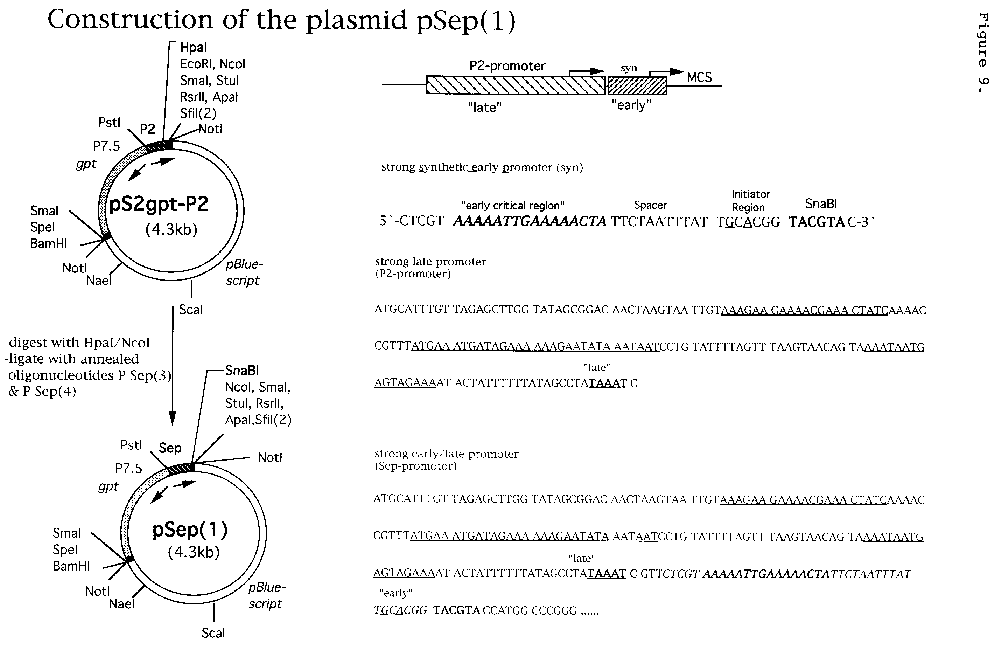

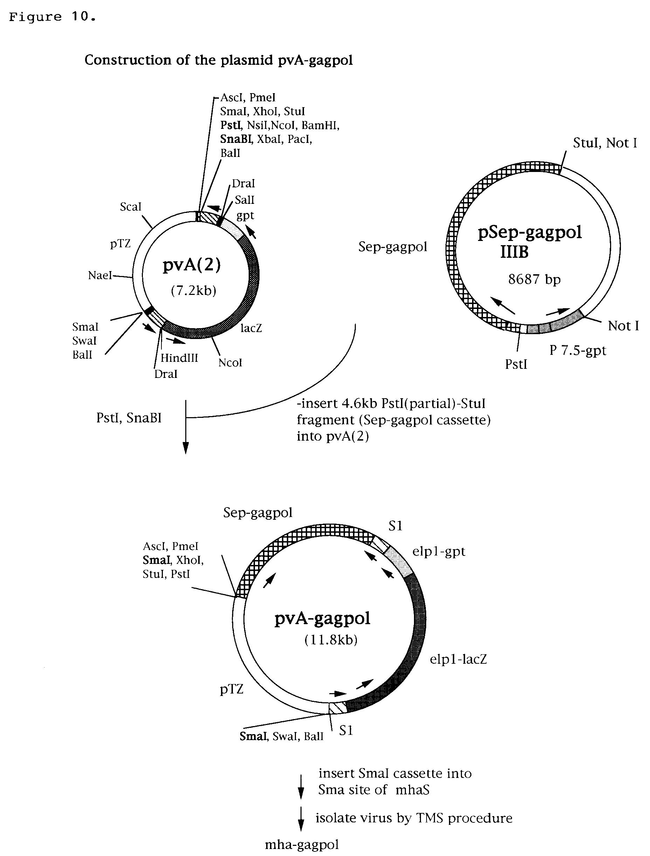

For this purpose a triple gene cassette consisting of the *lacZ* and the *gpt* genes (controlled by the elP1 promoter) and the HIV gagpol gene (controlled by the semi-synthetic early/late promoter Sep shown in Figure 9) was therefore inserted directly into the unique *Sma*I site of mhaS (Example 3). The construction of the plasmid pvA-gagpol carrying this triple gene cassette is shown in Figure 10. In this plasmid the foreign gene is adjacent to marker genes that are flanked by DNA segments. This arrangement allows, after integration of the triple gene cassette into the virus, transient marker stabilization and results, in a simple series of plaque purifications, in a final virus having no markers integrated.

To construct the virus mha-gagpol, the 9.0 kb triple gene cassette was excised from the plasmid pvA-gagpol as a *Sma*I fragment, inserted into the *Sma*I site of mhaS, and packaged in helpervirus (vaccinia MVA) infected cells according to Scheiflinger *et al., supra.* pvA-gagpol was constructed by cleaving plasmid pvA(2) (Example 1) with *Pst*I and *Sna*BI followed by ligation with the 4.6 kb Sep-gagpol fragment. Ligation and packaging was done in vaccinia MVA infected CV-1 cells as described by Scheiflinger *et al., supra.* The transient marker stabilization procedure was done as described in Example 3 except that the plasmid pvA-gagpol was used in the initial transfection of cells.

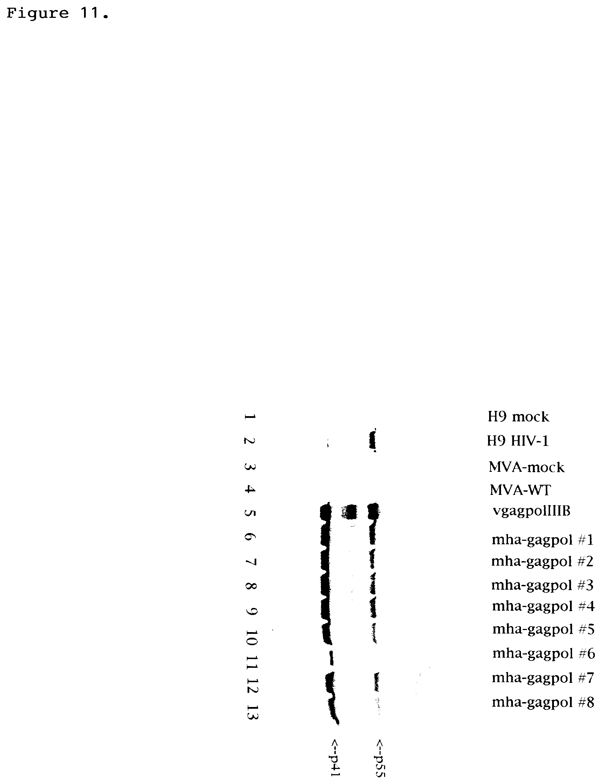

After growth for five days without selection the plaque assays were carried out as described in Example 3. The final stable viruses formed white plaques and were gpt-negative. Eight of the chimeric viruses (the mha-gagpol series) were further characterized. To check for expression small crude stocks were used to infect chicken embryo fibroblasts and the gagpol gene products were detected by Western blot (Figure 11).

The Western Blots were done essentially as described by Towbin *et al., supra.* For analysis of the gag-proteins the primary antibody was a sheep-anti p24 antibody (Accurate Chemical & Scientific Corporation; Westbury, NY, #BOK-D7320) used at a 1:500 dilution. The secondary antibody was a donkey-anti-sheep IgG/alkaline phosphatase conjugate (Serotec, Inc.) used at a 1:1000 dilution. For analysis of the pol-proteins the primary antibody was a monoclonal mouse anti-reverse transcriptase (HIV1IIIB) antibody (ABT, #9002, BIO-TRADE) used at a 1:1000 dilution (protein content 1ng/ul). The secondary antibody was a goat-anti-mouse IgG (H+L) alkaline phosphatase conjugate; (BIO-RAD #170-6520). The developing reagents (BCIP and NBT) and staining protocols were from Promega, Inc.

As shown in Figure 11, in comparison to the positive controls (HIV-1 infected H9 cells, lane 2, and vgagpolIIIB infected CV-1 cells, lane 5) the isolates mha-gagpol #1 to #8 showed the same p55 precursor and p41 protein bands (lanes 6-13). For unknown reasons isolate #6 showed a reduced level of p55 and p41. The smaller proteins products such as p24 were not visible on this specific blot because they run out of the gel.

On the basis of this screening experiment, three of the viruses showing the highest expression levels were grown to large scale, DNA was prepared and the genomic structures were analyzed by restriction digests and Southern blotting.

For further characterization the viruses are used for immunization experiments in rabbits, mice and chickens.

### Example 5. The thymidine kinase (tk) locus as insertion site for MVA-based chimeras: comparison of tk-positive and tk-negative MVA recombinants.

The tk-locus is a well characterized insertion site that allows stable integration of foreign genes in many vaccinia strains. The vaccinia tk-gene is not essential for growth of the virus in cell culture, at least for the Western Reserve strain (Mackett *et al., supra*). Viruses with inactivated tk-genes do, however, show decreased virulence and are highly attenuated in a mouse challenge model; growth in cell culture, however, is not impaired (Buller *et al., supra*).

To investigate the behaviour and the growth characteristics of tk-positive and tk-negative MVA strain derived viruses two types of viruses were constructed. In the first case the tk-gene was inactivated by gene insertion ('tk-negative MVA'), in the second case the endogenous tk-gene was inactivated, but, tk-gene activity was substituted by co-integration of the fowlpox virus (FPV) tk-gene ('FPV tk-positive MVA').

Unexpectedly the tk-negative MVA was difficult to handle, beginning with identification of plaques and plaque purification while FVP tk-positive MVA viruses were easy to purify and displayed better growth in cell culture and in vivo. These findings were the basis to construct the virus mtkS (see Example 6).

### Construction and characteristics of tk-negative MVA viruses.

For construction of the tk-negative MVA viruses the plasmid pTKgpt-F1sβ (Falkner *et al., J. Virol*. 62:1849 (1988)) was inserted into the tk-locus of the MVA strain by in-vivo recombination techniques in chicken embryo fibroblasts (CEF). The plasmid pTKgpt-F1sβ contains, between tk-gene flanking regions, the E. coli *gpt* and the *lacZ* gene. Recombinant viruses were identified by gpt-selection (Falkner and Moss, *supra*) and screening for lacZ expression (Chakrabarti *et al., supra*). Eighteen blue gpt-positive plaques were purified four times, and small virus stocks were grown and further characterized. Only ten plaques survived and could be fully purified.

For further characterization CEFs were infected with the small virus stocks, and after 72h total DNA was prepared, digested with *Xho*I and further analyzed by Southern blot. The immobilized fragments were hybridized to a vaccinia *tk*-gene probe (the plasmid pFS50 described in EP 0 538 046). As a size marker, the plasmid pTKgpt-Flsβ was digested with *Hind*III (pTKgpt-Flsβ, H) and with *Cla*I (pTKgpt-Flsβ, C).

The expected fragment of about 10.5kb containing the foreign gene and the tk-gene flank was detected. Most of the tk-negative MVA viruses, however, still contained the 5.3kb wild-type tk-gene fragment indicating that they were still contaminated by wild-type virus. Longer exposures of the blot showed that two isolates were free of wild-type virus.

This indicates that tk-negative MVA is difficult to handle and that tk-negative MVA grows less efficiently in cell culture than tk-positive MVA. This result is unexpected because the prior art describes unimpaired growth cell culture of tk-negative vaccinia. For tk-negative MVA more rigorous purification is required than with tk-positive vaccinia. Since tk-negative vaccinia viruses, in general, are highly attenuated in animals (Buller *et al., supra*) tk-negative MVA also is attenuated in animals. Since further attenuation of the already highly attenuated MVA is not desirable, the insertional inactivation of the tk-gene is not the procedure of first choice to express vaccine antigens.

### Construction and characteristics of FPV tk-positive MVA viruses

The *tk*-gene is located in the central part of the vaccinia genome and is a stable well characterized insertion site in several vaccinia strains. In an attempt to overcome the drawbacks described after using inactivational insertion into the tk-locus of the MVA strain, the fowlpox virus *tk*-gene was co-integrated into the vaccinia MVA tk-locus.

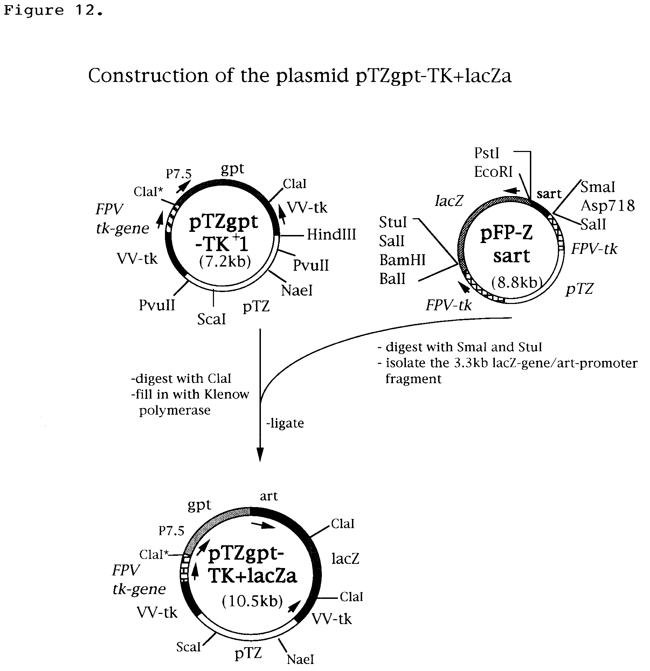

To construct the FPV tk-positive viruses, the plasmid pTZgpt-TK+lacZa was prepared by cleaving the *Sma*I-*Stu*I fragment containing the *lacZ* gene from the plasmid pFP-Zsart (EP 0 538 496) and inserting the fragment into pTZgpt-TK+1 (Scheiflinger *et al., supra.*) (Figure 12). The pTZgpt-TK+lacZa plasmid contains three foreign genes located between vaccinia *tk*-gene flanking regions, i.e. an intact FPV *tk*-gene and the *E. coli* genes *gpt* and *lacZ*. The latter two allow for gpt-selection and lacZ screening.

pTZgpt-TK+lacZa was inserted into the tk-locus of the MVA strain by *in vivo* recombination techniques. From ten initially isolated plaques, nine could be purified four times without difficulties. The nine plaques were named mTK#1.1, mTK#2.1, mTK#3.1, mTK#4.1, mTK#6.1, mTK#7.1, mTK#8.1, mTK#9.1 and mTK#10.1.

Viruses were characterized by Southern analyses using a vaccinia *tk*-gene probe (the plasmid pFS50 described in EP 0 538 496. As a size marker, the plasmid pTKgpt-F1sβ was digested with *Hind*III (pTKgpt-F1sβ, H) and with *Cla*I (pTKgpt-Flsβ, C). Characterization by Southern blotting revealed that the viruses showed the expected *Hind*III restriction and hybridization patterns of two fragments of 5.0 and 4.1 kb. Since this blot did not allow the co-detection of a possible wild-type contamination *Xho*I digests were also analyzed. The wild-type *Xho*I fragment containing the *tk*-gene was not visible even after long exposure times in the lanes of the mtk-viruses, indicating that the isolates were free of wild-type virus after four rounds of plaque purification. This was in contrast to the experience made with the tk-negative MVA viruses (see above).

Based on these results it was clear that the desired use of the vaccinia MVA tk-locus as insertion site is possible preferably by substituting *tk*-gene activity by a functional *tk*-gene of a related species. For the construction of the MVA virus mtkS, therefore, the FPV *tk*-gene was used to complement for the inactivated vaccinia *tk*-gene.

### Example 6. Construction of a modified host virus strain with a unique cloning site in the thymidine kinase gene, mtkS.

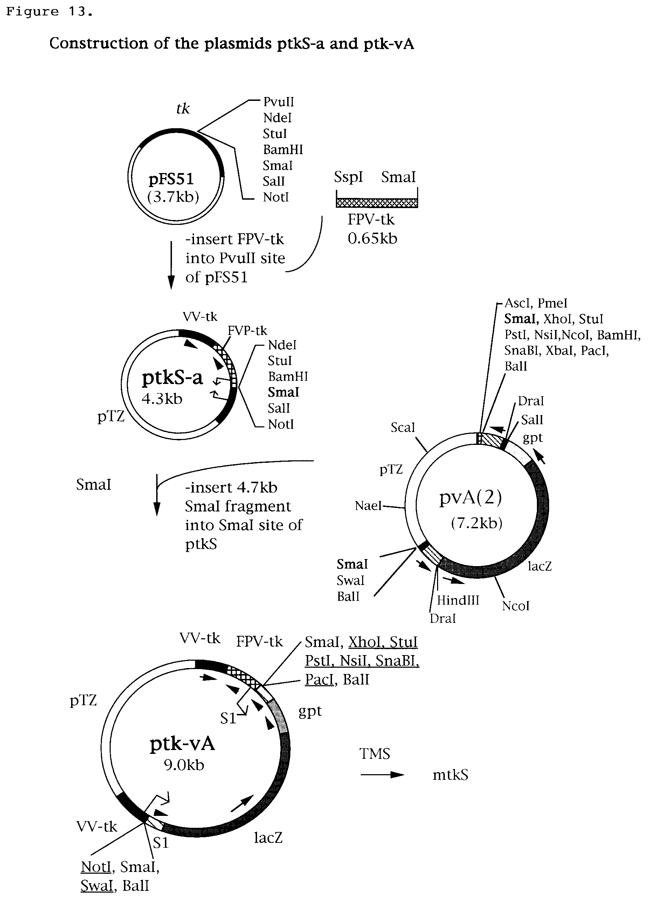

As shown in Example 5 insertional inactivation of the tk-locus of vaccinia MVA is of limited value unless tk-gene activity is substituted by a functional tk-gene of a related species. To construct an MVA-derived virus strain with a unique cloning site in the vaccinia tk-gene, therefore, plasmids were constructed in which the FPV *tk* gene is located between vaccinia tk-gene flanking regions (Figure 13). The plasmid ptkS-a was constructed by inserting the 0.65 kb *Ssp*I-*Sma*I fragment of pTKm, which contains the fowlpox virus *tk*-gene, into the *Pvu*II site of pFS51. (pTKm and pFS51 are described in EP 0 538 496. In ptkS-a, the vaccinia and FPV thymidine kinase genes are transcribed in opposite directions. The 4.7 kb *Sma*I fragment obtained from pvA(2) (see Example 1) containing the *gpt* and *lacZ* genes flanked by DNA segments S1, was ligated with ptkS linearized with *Sma*I to produce ptk-vA.

ptk-vA was used in a transient marker stabilization experiment to construct the virus mtkS that contains within the former vaccinia *tk*-gene a unique *Sma*I site and an FPV *tk*-gene to substitute for the lost endogenous tk-function. For this purpose, the plasmid ptk-vA was transfected into vaccinia MVA infected CV-1 cells and grown for five days without selection. The following procedure was performed as described in Example 3. The structure of the finally purified plaques was confirmed by restriction digests of the purified virus with several enzymes (such as *Sma*I, *Not*I, *Xho*I and *Hind*III) and subsequent agarose gel electrophoresis.

One of the isolates with the correct structure and normal growth was designated mtkS. This virus is used to insert gene cassettes by the direct molecular cloning technique into the viral *tk*-locus.

### Example 7. Construction of chimeric MVA vaccinia viruses expressing the HIV-1 gagpol gene in the tk-gene region (mtk-gagpol) by transient marker stabilization.

The HIV proteins are preferred candidates for vaccine preparations either in subunit or in live vaccines. In this example the expression of the gagpol open reading frame in the MVA strain mtkS is described. The HIV gagpol gene was inserted into the thymidine kinase locus of the virus mtkS (see Example 6) and may be used as a live vaccine to prime human immune response.

For this purpose a triple gene cassette consisting of the *lacZ* and the *gpt* genes (controlled by the elP1 promoter) and the HIV gagpol gene (controlled by the semi-synthetic early/late promoter Sep was inserted directly into the unique *Sma*I site of mtkS. The construction of the plasmid pvA-gagpol carrying this triple gene cassette is shown in Example 4, Figure 12. In this plasmid the foreign gene is adjacent to marker genes that are flanked by direct repeat DNA segments. Upon integration of the triple gene cassette into the virus this arrangement allows transient marker stabilization and, after a simple series of plaque purifications, results in a final virus having no integrated markers.

To construct the virus mtk-gagpol, the approximately 9kb triple gene cassette was excised as a *Sma*I fragment out of the plasmid pvA-gagpol, inserted into the *Sma*I site of mtkS and packaged in helpervirus (vaccinia MVA) infected CV-1 cells according to Scheiflinger *et al. supra*. The transient marker stabilization procedure was carried out essentially as in Example 3.

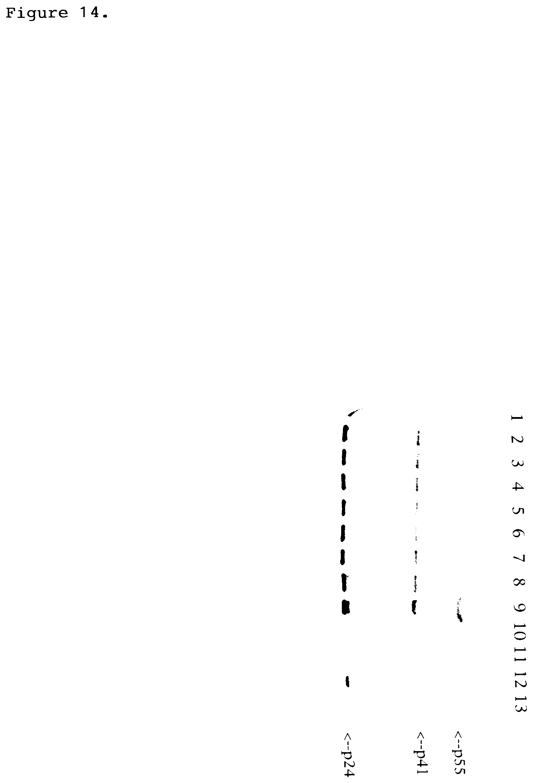

After growth for five days without selection the plaque assays were carried out as described in Example 3. The final stable viruses formed white plaques and were gpt-negative. Eight of the chimeric viruses (the mtk-gagpol series) were further characterized. To check for gagpol gene expression small crude stocks were used to infect chicken embryo fibroblasts and the gagpol gene products were detected in Western blots (Figure 14). The Western blots were done as described in Example 4. On the basis of this screening experiment, two virus isolates showing the highest expression levels were grown to large scale, DNA was prepared and the genomic structures were analyzed by PCR for the presence of the insert and for absence of contaminating wild-type virus.

For further characterization the viruses are used for immunization experiments in rabbits and mice.

### Example 8. Construction of chimeric MVA vaccinia viruses expressing the Hepatitis B Virus (HBV) large antigen gene in the tk-gene region, mtk-lAg, by transient marker stabilization.

Vaccinia or fowlpox viruses expressing HBV proteins are further candidates for live vaccines. In this example, the expression of the HBV large antigen (consisting of the preS1-S2 and S-Antigen) in the MVA strain mtkS is described. The large antigen gene is inserted into the thymidine kinase locus of the virus mtkS (see Example 6) and may be used as a live vaccine to prime human immune response.

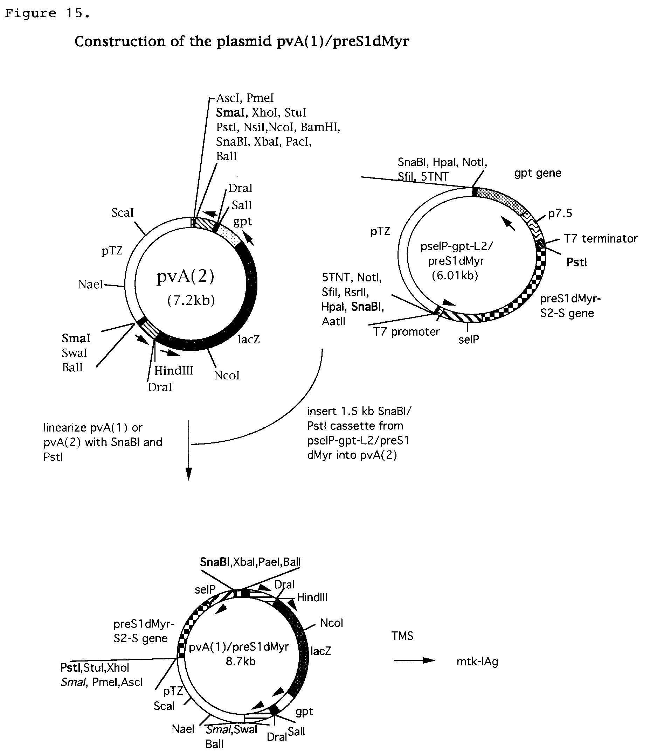

For this purpose, a triple gene cassette consisting of the *lacZ* and the *gpt* genes (controlled by the elP1 promoter) and the HBV large antigen gene (controlled by a synthetic early/late promoter) is therefore inserted directly into the unique *Sma*I site of mtkS. The construction of the plasmid pvA(1)/preS1dMyr carrying this triple gene cassette is shown in Figure 15. Briefly, the TMS cloning vector pvA(2) (Example 1) was linearized with the restriction enzymes *Pst*I and *Sna*BI. A 1.5 kb *Sna*BI/*Pst*I cassette containing a modified version of the Hepatitis B Virus preS1-S2-S gene fused to a synthetic vaccinia virus early late promoter sequence was isolated from plasmid pse1P-gpt-L2/preS1dMyr (EPA 0 637 631 A2). Vector and insert sequences were then ligated to produce the plasmid construct pvA(1)-preS1dMyr. In this plasmid, the foreign gene is adjacent to marker genes that are flanked by repeated DNA segments. This arrangement allows, after integration of the triple gene cassette into the virus, transient marker stabilization and results, in a simple series of plaque purifications, in a final virus expressing the large antigen with no integrated markers.

To construct the virus *mtk-lAg,* the triple gene cassette is excised as a *Sma*I fragment out of the plasmid pvA(1)-preS1dMyr, inserted into the *Sma*I site of mtkS and packaged in helper virus (vaccinia MVA) infected cells according to Scheiflinger *et al., supra*. After growth for five days without selection, the plaque assays are performed as described in Example 3. The final stable viruses form white plaques and are gpt-negative. Ten of the chimeric viruses (the mtk-1Ag series) are further characterized. To check for large antigen expression, small crude stocks are used to infect chicken embryo fibroblasts and the HBV large antigen gene products are detected in Western blots as described in (EPA 0 637 631 A2).

On the basis of this screening experiment, the viruses showing the highest expression levels are grown to a large scale, DNA is prepared and the genomic structures are analyzed by Southern blotting. For further characterization of the immunizing properties, the viruses are used for immunization experiments in rabbits, chickens, mice, and chimpanzees.

### Example 9. Construction of chimeric MVA vaccinia and fowlpox viruses expressing the Tick borne encephalitis (TBE) virus preM and E genes by transient marker stabilization.

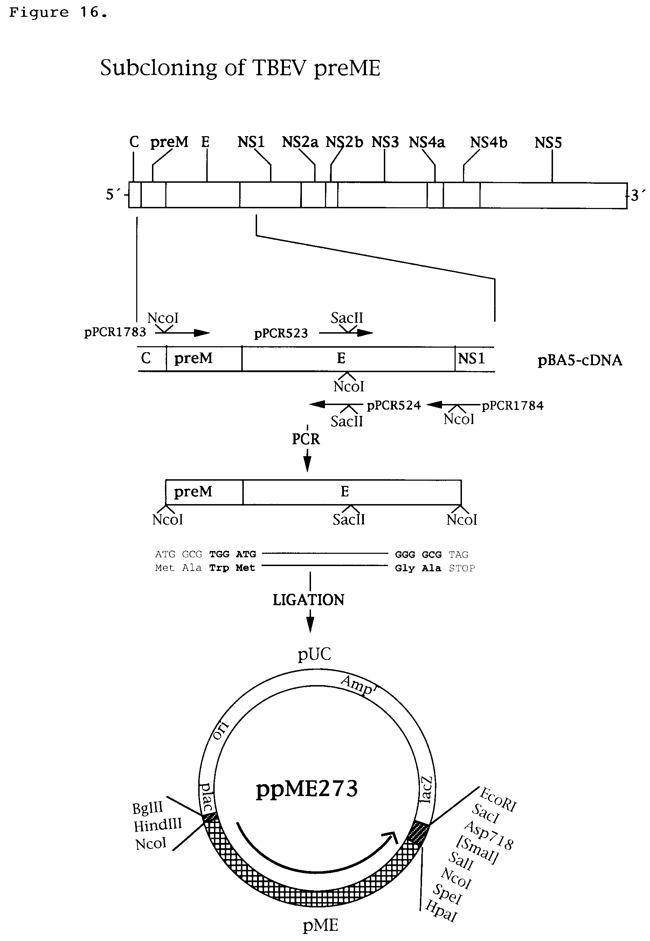

TBE virus belongs to the family of the flaviviridae. Its genome is a positive stranded RNA with a single open reading frame of about 11 kb coding for a polyprotein. This polyprotein is processed, either by cellular signalises or by virus encoded proteases, into the three viral structural proteins: the Core protein (C), the membrane protein (preM, M) and the envelope protein glycoprotein E (E) and a series of nonstructural proteins, NS1 through NS5 (Figure 16).

The structural glycoprotein E is the major antigen of TBE virus. Recombinant glycoprotein E of different flaviviruses was shown to be at least partially protective in animal models. Coexpression of preM and E of Yellow Fever Virus of Japanese Encephalitis Virus in vaccinia results in formation of extracellular particles which induce high levels of protection against lethal infections in mice. Mason *et al., Virol. 180*: 294-305 (1991); Konishi *et al., Virol. 188*: 714-720 (1992); Pincus *et al.,* Virol. *187*: 290-297 (1992).

Using PCR, the 2034 bp long preM-E coding region of TBEV (position 431-2458 according to the entry in the EMBL database - ID: TOGTBECG, AC: M27157; M21498) was amplified. The cDNA clone pBA5, containing part of the Core protein, the complete preM and E region and the 5' end of NS1 was used as template. Mandl *et al., Virol. 166*: 197-205 (1988) (Figure 16). The amplification was performed in two steps, destroying an internal *Nco*I restriction site (position 1648 - TOGTBECG) and introducing a *Sac*II site into the middle of the E region without changing the amino acid sequence. The PCR primers used for the amplification are pPCR523 (5'-CTG GCT CTG CCT TGG AAA CAT GAG GGC GCG CAA AAC TGG AC-3') (SEQ ID NO:23), pPCR524 (5'-CTG GAT CCA GTT TTG CGC GCC CTC ATG TTT CCA AGG CAG AGC CAG-3') (SEQ ID NO:24), pPCR1783 (5'-CTA CCA TGG CGT GGA TGA GCT GGT TGC TGG TC-3') (SEQ ID NO:25) and pPCR1784 (5'-CAG TCG ACC ATG GAC TAG TTA ACT ACG CCC CCA CTC CAA GGG TCA-3') (SEQ ID NO:26).

The two PCR fragments were assembled in a pUC vector (ppME273 - Figure 16). The sequence of the resulting *Nco*I cassette was confirmed by sequence analysis. The deduced amino acid sequence (678 amino acids) is identical to that predicted from pBA5. In order to introduce a *Nco*I site, two amino acids, a methionine and alanine were added in front of the trp-met at the 5'-end. The last amino acids gly-ala at the 3'-end (position 2458 - TOGTBECG) are followed by a TAG stop codon within the *Nco*I cassette.

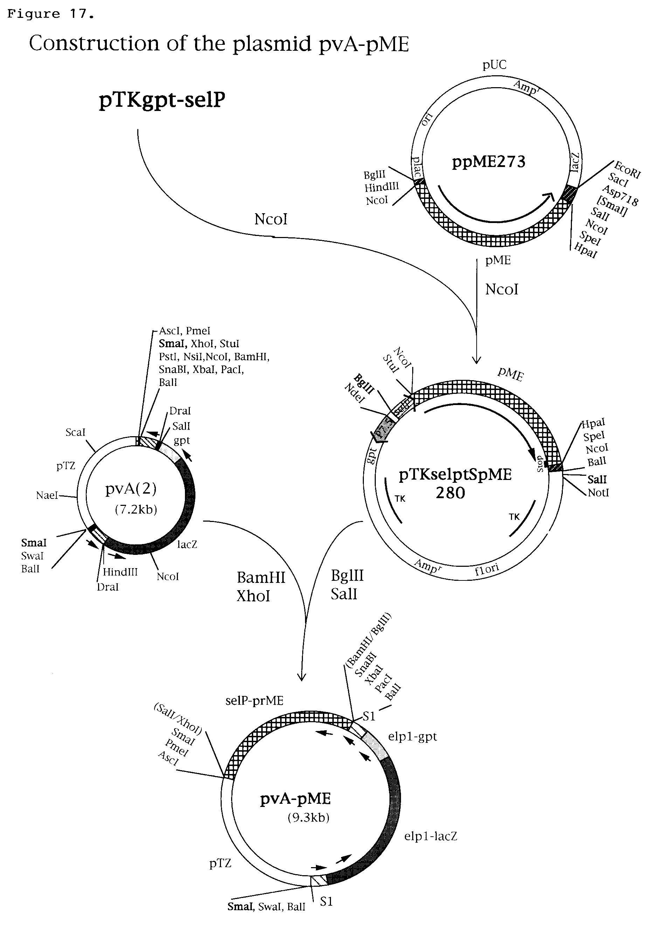

To construct a chimeric virus, the pME-*Nco*I cassette (2048 bp) from plasmid ppME273 was first subcloned into the vaccinia vector pTKgpt-selP behind a synthetic early/late promoter (selP). pTKgpt-selP is a vaccinia virus insertion plasmid with a dominant selection marker and a strong synthetic early/late poxvirus promoter (Davison *et al, J. Mol. Biol.* 210: 749-769 (1989)). It was constructed by annealing the oligonucleotides oselP1, (5' TATGAGATCT AAAAATTGAA ATTTTATTTT TTTTTTTTGG AATATAAATA AGGCCTCCAT GGCCATAATT AATTAATTTT TCTG 3') (SEQ ID NO:27) and oselP2, (5'TCGACAGAAA AATTAATTAA TTATGGCCAT GGAGGCCTTA TTTATATTCC AAAAAAAAAA AATAAAATTT CAATTTTTAG ATCTCA 3') (SEQ ID NO:28). The annealed oligonucleotides are inserted into the *Nde*I-*Sal*I sites of pFSgpt (EP 0 538 496). In the final step, the selP-pME cassette from pTKselptspME280 was excised as a *Bgl*II-*Sal*I fragment and ligated into the *Xho*I, *Bam*HI site of pvA(2) resulting in the plasmid pvA-pME (Figure 17).

To construct chimeric viruses, a triple gene cassette consisting of the *lacZ* and the *gpt* genes (controlled by the elP1 promoter) and the TBE preME gene is therefore inserted directly into the unique *Sma*I sites of either mhaS (Example 3), mtkS (Example 6) and f-tkS (Example 10). The triple gene cassette is excised as a *Sma*I fragment out of the plasmid pvA-pME, inserted into the *Sma*I sites of the respective viruses and packaged in helpervirus infected cells according to Scheiflinger *et al., supra*. After growth for five days without selection the plaque assays are performed as described in Example 3. The final stable viruses from white plaques and are gpt-negative.

Thus, for example, to produce the virus mtk-prME viral DNA of the virus mtkS (Example 6) is cut with *Sma*I and vector arms are ligated with the triple gene cassette excised from plasmid pvA-pME. As another alternative, to produce the virus ftk-prME viral DNA of the virus ftkS (Example 10) is cut with *Sma*I and vector arms are ligated with the triple gene cassette excised from plasmid pvA-pME. In each case, ligation and packaging are performed using fowlpox virus infected CV-1 cells as described by Scheiflinger *et al., supra*. The transient marker stabilization procedure is performed as described in Example 3 except that the triple gene cassette excised from the plasmid pvA-pME is used in the ligation experiment with the viral vector arms.

To check for preME gene expression, small crude stocks are used to infect chicken embryo fibroblasts and the gene product is detected in Western blots. Western blots analyses were performed essentially as described by Towbin *et al. supra*. The primary antibody was a monoclonal anti-TBEV glycoE antibody isolated from mice immunized with recombinant E-protein from *E. coli* (IMMUNO #130.L3). The cell culture supernatant was used in a 1:50 dilution. The secondary antibody was a goat-anti-mouse IgG coupled with alkaline phosphatase (BioRad, Inc. #170-6520) used in a 1:1000 dilution. The reagents (BCIP and NBT) and staining protocols are from Promega, Inc. On the basis of this screening experiment, the viruses showing the highest expression levels are grown to a large scale, DNA is prepared and the genomic structures are analyzed by Southern blotting.

For further characterization, the viruses are used for immunization and challenge experiments in mice.

### Example 10. Construction of a modified fowlpox virus strain with a unique cloning sites, f-tkS, by transient marker stabilization.

As discussed above, Fowlpox virus (FPV), as vaccinia MVA, are viruses that are difficult to handle. The use of selection markers has simplified the process of obtaining chimeric virus, but viruses expressing foreign genes alone in the absence of a marker function are still difficult to obtain. The transient marker stabilization technique was therefore applied to construct chimeric FPV viruses.

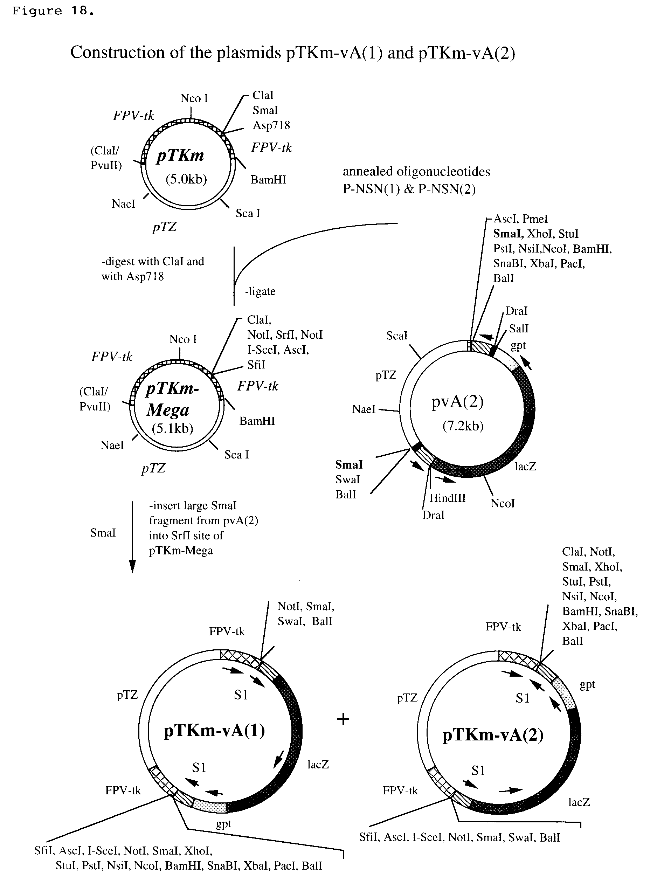

The first step was to construct a host virus strain (f-tkS) that has unique cloning sites in a stable central part of the genome. To obtain this virus, the plasmids pTKm-vA(1) and pTKm-vA(2) were constructed (Figure 18). Briefly, the annealed oligonucleotides P-NSN(1), (5'-CGATGCGGCC GCGCCCGGGC GCGGCCGCTA GGGATAACAG GGTAATGGCG CGCCGGCCAT ATAGGCCA-3')(SEQ ID NO:29), and P-NSN(2), (5'-GTACTGGCCT ATATGGCCGG CGCGCCATTA CCCTGTTATC CCTAGCGGCC GCGCCCGGGC GCGGCCGCAT-3')(SEQ ID NO:30), were inserted between the *Cla*I and *Asp*718 sites of pTKm resulting in pTKm-Mega. The large *Sma*I fragment containing the double marker gene cassette was then inserted into the *Srf*I site of pTKm-Mega resulting in pTKm-vA(1) and pTKm-vA(2).

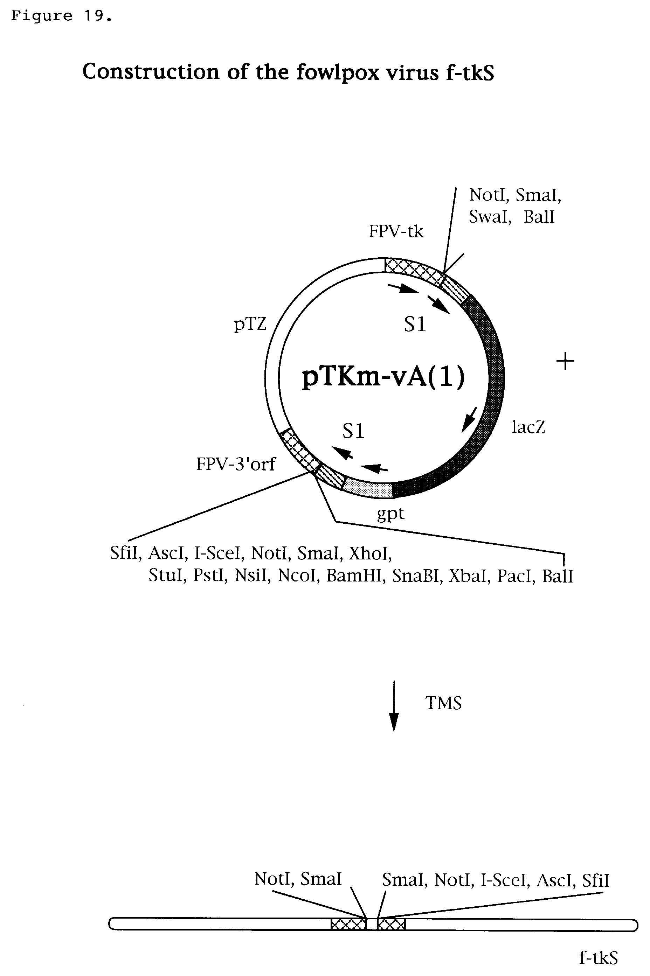

These plasmids are used in a transient marker stabilization experiment to construct the virus f-tkS that contains unique *Not*I and I-*Sce*I sites downstream of the *tk*-gene (Figure 19). For this purpose, the plasmid pTKm-vA(1) was transfected into FPV-infected chicken embryo fibroblasts (as described in the transient marker stabilization procedure in Example 3 except that chicken embryo fibroblasts were used instead of CV-1 cells) and grown for five days without selection. Plaque assays were performed as described in Example 3.

The structure of the finally purified white plaques is confirmed by restriction digests of the purified virus with several enzymes (such as *Sma*I, *Not*I, I-*Sce*I, AscI, SfiI, *Xba*I and *Hpa*I) and subsequent Southern analyses. One of the isolates with the correct structure and normal growth is designated f-tkS. This new fowlpox virus host strain is used to insert gene cassettes by direct molecular cloning and the TMS technique (see Example 11 and 12).

### Example 11. Construction of the chimeric fowlpox virus expressing the HIV gagpol gene, ftk-gagpol, by transient marker stabilization.

Fowlpox viruses expressing HIV proteins are further candidates for live vaccines. In this example the expression of the HIV gagpol gene in the FPV strain f-tkS is described. The gagpol gene is inserted between the *Srf*I sites of the virus f-tkS (see Example 10) and may be used as a live vaccine to prime human immune response.

For this purpose a triple gene cassette consisting of the *lacZ* and the *gpt* genes and the HIV gagpol gene is therefore inserted directly between the *Sma*I sites of f-tkS. The plasmid pvA-gagpol carrying this triple gene cassette is shown in Figure 10. In this plasmid the foreign gene is adjacent to marker genes that are flanked by repeated DNA segments. This arrangement allows, after integration of the triple gene cassette into the virus, transient marker stabilization and results, in a simple series of plaque purifications, in a final virus expressing the large antigen with no integrated markers.

To construct the virus ftk-gagpol, the triple gene cassette is excised as a *Sma*I fragment out of the plasmid pvA-gagpol, inserted between the SmaI sites of f-tkS and packaged in helpervirus (FPV) infected cells according to Scheiflinger *et al. supra*. After growth for five days without selection the plaque assays are done as described in Example 3. The final stable viruses form white plaques and are gpt-negative. Ten of the chimeric viruses (the f-tkgagpol-series) are further characterized. To check for gagpol antigen expression small crude stocks are used to infect chicken embryo fibroblasts and the gene products are detected in Western blots.

For analysis of the *gag*-proteins, the primary antibody was a sheep anti-p24 antibody (Accurate Chemical & Scientific Corporation, Westbury, NY, #BOK-D7320) used at a 1:500 dilution. The secondary antibody was a donkey anti-sheep IgG coupled with alkaline phosphatase (obtained from Serotec, Inc.) used at a 1:1000 dilution. For analysis of the *pol* proteins, the primary antibody was a monoclonal, mouse anti-reverse transcriptase (HIV-1_{IIIB}) antibody (ABT, #9002, BIO-TRADE) used at a 1:1000 dilution (protein content 1 ng/ul). The secondary antibody was a goat antimouse IgG (H+L) alkaline phosphatase conjugate (BIO-RAD #170-6520).

On the basis of this screening experiment, the viruses showing the highest expression levels are grown to large scale, DNA is prepared and the genomic structures are analyzed by Southern blotting. For further characterization the viruses are used for immunization experiments in rabbits, chickens and mice.

### Example 12 Construction of chimeric MVA vaccinia viruses co-expressing the HIV-1 env gene of the Chiang Mai (CM) strain and the human gamma-interferon gene (mtk-e/IFN) in the tk-gene region by transient marker stabilization.

HIV proteins are preferred candidates for vaccine preparations either in subunit or in live vaccines. This example describes the co-expression of the HIV-1 Chiang Mai *env* gene and the human gamma-interferon (γ-IFN) gene in the MVA strain mtkS. Both genes are inserted into the thymidine kinase locus of the virus mtkS (see Example 6 ) and are used as a candidate live vaccine to prime human immune response.

A truncated form of the HIV-1 *gp160* gene of the HIV CM235 strain has been published under the identification number ID.: HIV1CM235 in the EMBL and gene bank data base. This *env* gene lacks the sequences coding for the carboxyterminal aminoacids. To obtain a complete gene corresponding to the published HIV-1 sequences of the MN strain (Gurgo *et al., Virol.* 164: 531-536 (1988) these missing sequences were reconstructed by molecular cloning techniques.

Briefly, analysis of the plasmid pCMenv (containing the *gp160* gene of the virus CM235 cloned as an EcoRI-SalI fragment into the pBLUESCRIPT vector (Stratagene, Inc.) showed that the carboxy-terminal 9 aminoacids were missing. The MN type envelope protein was used to replace the missing amino acids. To accomplish this, a PCR primer was synthesized that was homologous to the 3'end of CM235 and that also contained an additional 27 nucleotides coding for the missing amino acids and the translation termination signal TAA. To facilitate further cloning procedures a *Sal*I site and *Nco*I site were included. PCR was carried out with the PCR primer sprm.CMenv2148, as 5'-primer, and the 3'-PCR primer pPCR.CMenv2211, using the cDNA plasmid pCM-env1 as template. The sequence of sprm.CMenv2148 was (5'-ATT CTC CAC ATA CCT AGA AGA ATC AGA CAG GGC TTG GAA AGG GCT TTG CTA TAA GTCGACCATGGG-3') (SEQ ID NO:31); the sequence of pPCRCMenv2211 was (5'-CCCATGGTCG ACTTATAGCA AAGCCCTTTC CAAGCCCTGT CTGATTCTTC TAGGTATGTG GAGAAT-3') (SEQ ID NO:32). The PCR product was purified on QIAquick-spin PCR purification columns (Qiagen, Inc., Chatsworth,CA, Cat. No. 28104), digested with the restriction enzymes *Eco47*III and *Sal*I and ligated into the plasmid pCM-env1 that had previously been digested with the same enzymes, resulting in the plasmid pBlue4-CM235r.

pBlue4-CM235r was then digested with *Sma*I and *Apa*I to release the *env* gene which was then ligated between the *SnaB*I and *Apa*I sites of pSep(1), a vector containing a strong poxvirus early/late promoter (see Figure 9) resulting in pSep-eCM1. A multiple cloning site, consisting of the annealed oligonucleotides oSalApa1 (5'-TCG ACG AAT TCG TTT AAA CCC ATG GGC GGC CGC GCC CGG GCG GGC C-3') (SEQ ID NO:33) and oSalApa2 (5'-CGC CCG GGC GCG GCC GCC CAT GGG TTT AAA CGA ATT CG-3') (SEQ ID NO:34) was then inserted between the unique *Sal*I and *Apa*I sites of pSep-eCM1, resulting in the plasmid pSep-eCM2.

The human gamma-interferon (γ-IFN) gene under the control of the synthetic early/late promoter elP1 (see Example 1) was co-inserted into the plasmid pSep-eCM2 resulting in the plasmid pSep-eCM/IFN . The human gene for interferon-γ was purchased from British Biotechnology (R&D Systems, Minneapolis, MN, Cat. No. BBG6) as a cDNA clone in pUC18. The completed IFN gene was subcloned as an *Eco*RI, *Bam*HI fragment into a derivative of pUC19, containing a *RcaINcoI* site at the 5'-end of the gene resulting in the plasmid pUC19 p-IFN-250. The IFN signal sequence missing in the original BBG6 and pIFN250 construct was chemically synthesized in both strands with *RcaI SplI* compatible ends (oligonucleotides IFNg.sig-us2735, (5'-CATG AAA TAT ACA AGT TAT ATC TTG GCT TTT CAG CTC TGC ATC GTT TTG GGT TCT CTT GGC TGT TAC TGC CAG GAC CC-3') (SEQ ID NO:35) and IFNg.sig-ls2734, (5'-GTACGG GTC CTG GCA GTA ACA GCC AAG AGA ACC CAA AAC GAT GCA GAG CTG AAA AGC CAA GAT ATA ACT TGT ATA TTT-3') (SEQ ID NO:36) and cloned into the RcaI and SplI sites of pIFN250 resulting in plasmid pSIFN-356. To adopt this gene to the requirements of the plasmid pelP1-gpt it was amplified by PCR using the pSIFN-356 pUC19-IFN plasmid as template. The 5'-primer pPCR.IFN-5'-2761 (5'-AAGGCCTCATG AAA TAT ACA AGT TAT ATC TTG GCT-3') (SEQ ID NO:37), pPCR.IFN-5'-2278, (5'-AAG GCC TGC ATG CAG GAC CCG TAC GTA-3') (SEQ ID NO:38) introduced a *StuI* and *RcaISphI* site at the 5'-end. The ATG translation initiation signal is part of the *RcaISphI* site. The 3'-primer used sprm.pUC 1225, (5'-GTT TTC CCA GTC ACG ACG TTGGTG TGG AAT TGT GAG CGG A-3') (SEQ ID NO:39) anneals to the *lacZ* promotor region in pUC vectors. The overall structure of the resulting gene is: - *StuI RcaI* (ATG)-IFN-γ signal-IFN-γ-(166 amino acids)(TAG)translation Stop-*Bam*HI-*Xba*I-*Sal*I.

The resulting PCR fragment was cloned as a *Stu*I, *Sal*I fragment into pelP1-gpt that had previously been digested with *Hpa*I and *Sal*I. The gpt fragment of the vector is thereby substituted by the IFN gene, placing the elP promotor upstream of the IFN-gene and producing plasmid pelP1-IFN-336.

The *Nde*I-*Bam*HI gene fragment containing the P7.5-gpt gene cassette in pSep-eCM2 was substituted by the *Nde*I-*BamH*I gene fragment containing the elP1-IFN gene cassette (obtained from the plasmid pelP1-IFN), resulting in pSep-eCM/IFN. To obtain a construct including the *env* gene, the IFN gene and the double marker genes that are required for the transient selection procedure, the plasmids ptk-eCM/IFNa and ptk-eCM/IFNb were constructed. The *Not*I fragment consisting of the gene cassettes Sep-gp160CM and elP1-IFN was inserted into the unique *Not*I site of ptk-vA (described in Example 6), resulting in the plasmids ptk-eCM/IFNa and ptk-eCM/IFNb.

To construct the virus mtk-e/IFN by direct molecular cloning the *env* gene, the IFN gene, and the double marker genes were inserted as a *Sma*I fragment (obtained from the plasmid ptk-eCM/IFNb) into the unique *Sma*I site of mtkS (mtkS is described in Example 8). Viral DNA of the virus mtkS (Example 8) is cut with *Sma*I and vector arms are ligated with the approx. 9.0 kb gene cassette excised from plasmid pSep-eCM/IFN. Ligation and packaging is done in vaccinia MVA infected CV-1 cells as described by Scheiflinger *et al., supra*. Alternatively, the plasmids ptk-eCM/IFNa and ptk-eCM/IFNb may be used in recombination experiments to obtain the desired viruses. The transient marker stabilization procedure is done as described in Example 3.

In the aforementioned constructs the foreign genes are adjacent to marker genes that are flanked by DNA segments. This arrangement allows transient marker stabilization to occur, after integration of the foreign genes into the virus, and after a simple series of plaque purifications results in a final virus having no integrated markers.

The final stable viruses form white plaques and are gpt-negative. Ten of the chimeric viruses are further characterized. To check for *env* and IFN gene expression small crude stocks are used to infect chicken embryo fibroblasts and the env and IFN gene products are detected in Western blots. The Western blots to detect gp160 expression were done essentially as described by Towbin *et al., supra*. The primary antibody was anti-HIV-type E monoclonal antibody (Muster et al., J. Virol., 67(11): 6642-6647, 1993) The hybridoma cell culture supernatant was used in a 1:20 dilution. The secondary antibody was a goat-anti-human IgG coupled with alkaline phosphatase (BioRad, Inc. #172-1004) used at a 1:1000 dilution. The developing reagents (BCIP and NBT) and staining protocols are from Promega, Inc.

For the Western blots to detect IFN-γ expression the primary antibody was a rabbit anti-human IFN-g antiserum (obtained from Genzyme, Inc. cat. No. IP500) used at a 1:500 dilution. The secondary antibody was a goat-anti-rabbit IgG coupled with alkaline phosphatase (BioRad, Inc.) used at a 1:1000 dilution. On the basis of this screening experiment, three virus isolates showing the highest expression levels are grown to large scale, DNA is prepared and the genomic structures are analyzed by PCR for the presence of the insert and for absence of contaminating wild-type virus.

For further characterization the viruses are used in immunization experiments in rabbits, chickens and mice.

### Example 13 . Construction of chimeric MVA vaccinia viruses co-expressing the HIV-1 env gene of the Chiang Mai (CM) strain and the human interleukin 6 gene (mtk-eIL6) in the tk-gene region by transient marker stabilization.

The HIV proteins are preferred candidates for vaccine preparations either in subunit or in live vaccines. This example describes the co-expression of the HIV-1 Chiang Mai env gene and the interleukin 6 (IL6) gene in the MVA strain mtkS. Both genes are inserted into the thymidine kinase locus of the virus mtkS (Example 6) which is then used as a candidate live vaccine to prime human immune response.

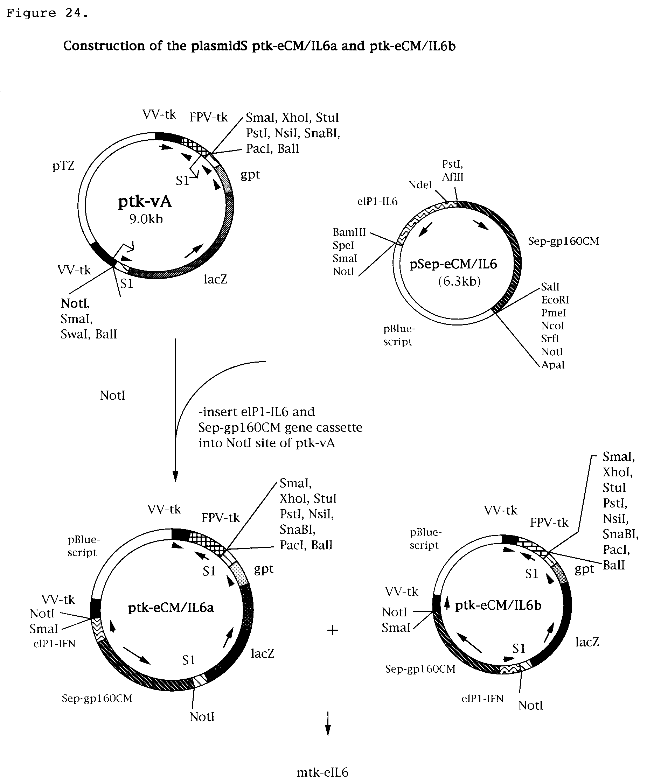

The HIV-1 gp160 gene of the CM235 strain (Example 12) was inserted into the vector pSep(1) (Example 4), resulting in the plasmids pSep-eCM1 and pSep-eCM2 that contain the Sep-env gene cassette (Example 12). The human interleukin 6 (IL6) gene controlled by the synthetic early/late promoter elP1 (see Example 1) ). was co-inserted into the plasmid pSep-eCM2, resulting in the plasmid pSep-eCM/IL6 (Figure 24).

To construct pSep-eCM2, a cDNA clone containing the human gene for interleukin 6 in pUC18 was purchased from British Biotechnology (R&D Systems Catalog # BBG17). The gene was subcloned as an *EcoR*I, *BamH*I fragment into the plasmid pUC19, resulting in pIL6-343. This IL6 clone lacks the natural signal sequence which was substituted as follows: A newly synthesized 5'-primer pPCR.IL6sig2747 (5'-AAGGCCTC ATG AAC TCC TTC TCC ACA AGC GCC TTC GGT CCA GTT GCC TTC TCC CTG GGG CTG CTC CTG GTG TTG CCT GCT GCC TTC CCT GCC CCA GTA CCC CCA GGA GAA GAT TC-3') (SEQ ID NO:40) introduced a *StuI* and *RcaI* site and the signal sequence missing in BBG17 and pIL6-343 at the 5'-end. The ATG translation initiation signal is part of the RcaI site. The 3'-primer used sprm.pUC1761, (5'-GTT TTC CCA GTC ACG ACG TTG-3') (SEQ ID NO:41) anneals to the *lacZ* region in pUC vectors.

The completed gene was subcloned as an *EcoR*I, *BamH*I fragment into the plasmid pUC19, resulting in pUC19-IL6. To adopt this gene to the requirements of the vector pelP1-gpt it was amplified by PCR using the pUC19-IL6 plasmid as template. The 5'-primer (pPCR.IL6-(5'-2278, 5'-AAG GCC TGC ATG CCG GTA CCC CCA GGA-3') (SEQ ID NO:42) introduced a *Stu*I and *Sph*I site at the 5'-end. The ATG translation initiation signal is part of the *Sph*I site. The 3'-primer used sprm.pUC 1225, described in Example 12, is a primer that anneals to the *lac* promotor region in pUC vectors. The overall structure of the gene is: *Stu*I-*Rca*I (ATG)-IL6signal-IL6-(212 amino acids)-(TAG)translation Stop-*BamHI-SmaI*.

The resulting PCR fragment was cloned as a *Stu*I and *Sma*I fragment into pelP1-gpt (see Example 1) that had previously been cleaved with *Hpa*I and *SmaI*. The gpt fragment of the vector is substituted by the IL6 gene thereby placing the elP1 promotor upstream of the gene. The resulting plasmid was named pelP1-IL6-358. The *Nde*I-*BamH*I gene fragment containing the P7.5-gpt gene cassette in pSep-eCM2 was substituted by the *Nde*I*-BamH*I gene fragment containing the elP1-IL6 gene cassette (obtained from the plasmid pelP1-IL6-358), thereby producing pSep-eCM/IL6. The plasmids ptk-eCM/IL6a and ptk-eCM/IL6b were constructed to contain the *env* gene, the IL6 gene and the double marker genes (Figure 24). The *Not*I fragment consisting of the gene cassettes Sep-gp160CM and elP1-IL6 was inserted into the unique *Not*I site of ptk-vA (Example 6 ) resulting in the plasmids ptk-eCM/IL6a and ptk-eCM/IL6b.

To construct the virus mtk-eIL6, the *env* gene, the IL6 gene, and the double marker genes are inserted as *Sma*I fragment (obtained from the plasmid ptk-eCM/IL6b) into the unique *Sma*I site of mtkS (Example 6). Viral DNA of the virus mtkS (Example 8) is then cut with *Sma*I and vector arms are ligated with the approx. 9.0 kb gene cassette excised from plasmid pSep-eCM/IL6. Ligation and packaging is done in vaccinia MVA infected CV-1 cells as described by Scheiflinger *et al., supra*. The transient marker stabilisation procedure is done as described in Example 3. Alternatively, the plasmid ptk-eCM/IL6b may be used in a recombination experiment with MVA wildtype virus to obtain the desired recombinant viruses.

In the aforementioned constructs the foreign genes are adjacent to marker genes that are flanked by DNA segments. This arrangement allows transient marker stabilization to occur, after integration of the foreign genes into the virus, and after a simple series of plaque purifications results in a final virus having no integrated markers.

The final stable viruses form white plaques and are gpt-negative. Ten of the chimeric viruses are further characterized. To check for *env* and IL6 gene expression small crude stocks are used to infect chicken embryo fibroblasts and the *env* and IL6 gene products are detected in Western blots. The Western blots are done as described in Example 12, except that a rabbit-anti-human IL-6 antiserum (Genyme, Inc.) in a 1:500 dilution is used to detect IL-6. On the basis of this screening experiment, three virus isolates showing the highest expression levels are grown to large scale, DNA is prepared and the genomic structures are analyzed by PCR for the presence of the insert and for absence of contaminating wild-type virus.

For further characterization the viruses are used in immunization experiments in rabbits, chickens and mice.

### Example 14. Construction of chimeric MVA vaccinia viruses co-expressing the human gamma-interferon (γ-IFN) gene and the HIV-1 genes gagpol and env (m-gpe-IFN).

The HIV proteins are preferred candidates for vaccine preparations either in subunit or in live vaccines. This example describes the co-expression of the HIV-1 *env* (gp160) and *gagpol* genes together with the human γ-interferon gene in the MVA strain. The *env* gene is derived from the Chang Mai strain (Example 12) and the *gagpol* genes (Example 4) are derived from the HIV-1 IIIB strain. The *env* and the IFN genes are inserted in the viral thymidine kinase locus while the *gagpol* genes are located in the hemeagglutinin locus. The virus is constructed by genetic crosses of the virus mtk-eCM/IFN (Example 12) and the virus mha-gagpol (Example 4).

The virus m-gpe-IEN is used as a candidate AIDS live vaccine to prime human immune response, preferably human T-cell response. To demonstrate its utility in an animal model chickens, rabbits and mice are immunized with this virus and B and T cell response in measured.

To construct the virus m-gpe-IFN, primary chicken embryo fibroblasts are co-infected with 1 pfu/cell of the viruses mha-gagpol and mtk-eCM/IFN. After three days of growth a crude viral stock is prepared from the cells, followed by plaque purification. Co-infection of different vaccinia viruses results in high levels of recombinants (Ball *J.* *Virol.* 61: 1788-1795 (1987)). Double recombinant viruses are identified by screening of single plaques by PCR and subsequent Western Blotting (as described in Example 12 ). Viruses positive for gp160 and *gagpol* expression are further characterized by restriction analysis and Southern blotting.

### Example 15 Construction of chimeric MVA vaccinia viruses co-expressing the human interleukin 6 (IL6) gene and the HIV-1 genes gagpol and env (m-gpe-IL6).

HIV proteins are preferred candidates for vaccine preparations either in subunit or in live vaccines. This example describes the co-expression of the HIV-1 *env* (gp160) and *gagpol* genes together with the human interleukin 6 gene in the MVA strain. The *env* gene is derived from the Chiang Mai strain (Example 12 ) and the *gagpol* genes (Example 4 ) are derived from the HIV-1 IIIB strain. The *env* and the IL-6 genes are inserted in the viral thymidine kinase locus while the *gagpol* genes are located in the hemeagglutinin locus. The virus is constructed by genetic crosses of the virus mtk-eCM/IL-6 (Example 13 ) and the virus mha-gagpol (Example 4 ).

The virus m-gpe-IL-6 is used as a candidate AIDS live vaccine to prime human immune response, preferably human T-cell response. To demonstrate its utility in an animal model chickens, rabbits and mice are immunized with this virus and B and T cell response in measured.

To construct the virus m-gpe-IL-6, primary chicken embryo fibroblasts are co-infected with 1 pfu/cell of the viruses mha-gagpol and mtk-eCM/IL-6. After three days of growth a crude viral stock is prepared from the cells, followed by plaque purification. Double recombinant viruses are identified by screening of single plaques by PCR and subsequent Western Blotting (as described in Example 12 ). Viruses positive for gp160 and *gagpol* expression are further characterized by restriction analysis and Southern blotting.

### Example 16 Construction of chimeric MVA vaccinia viruses co-expressing the HIV-1 genes gagpol and env (m-gpe) by the transient marker stabilisation (TMS) technique.

HIV proteins are preferred candidates for vaccine preparations either in subunit or in live vaccines. This example describes the co-expression of the HIV-1 *env* (gp160) and *gagpol* genes in the MVA strain is described. In this construct, termed m-gpe, no immunomodulating co-factors are included. This virus can serve as an immunogen to elicit an unmodified response to *gag*, *pol* and *env* antigens, or as a control to contrast the effects of the co-expressed immunomodulators (see Examples 12-15 ). It is constructed by the TMS technique and has no marker genes integrated.

The *env* gene is derived from the Chiang Mai strain (Example 12 ) and the *gagpol* genes (see also Example 4) are derived from the HIV-1 IIIB strain. The *env* gene is inserted in the viral thymidine kinase locus while the *gagpol* genes are located in the hemeagglutinin locus. The virus is constructed by genetic crosses of the virus mtk-CMenv (constructed as described below) and the virus mha-gagpol (described in Example 4).

The virus m-gpe, which is the crossing product of the viruses mtk-CMenv and mha-gagpol, is used as a candidate AIDS live vaccine to prime human immune response. To demonstrate its utility in an animal model chickens, rabbits and mice are immunized with this virus and B and T cell response in measured.

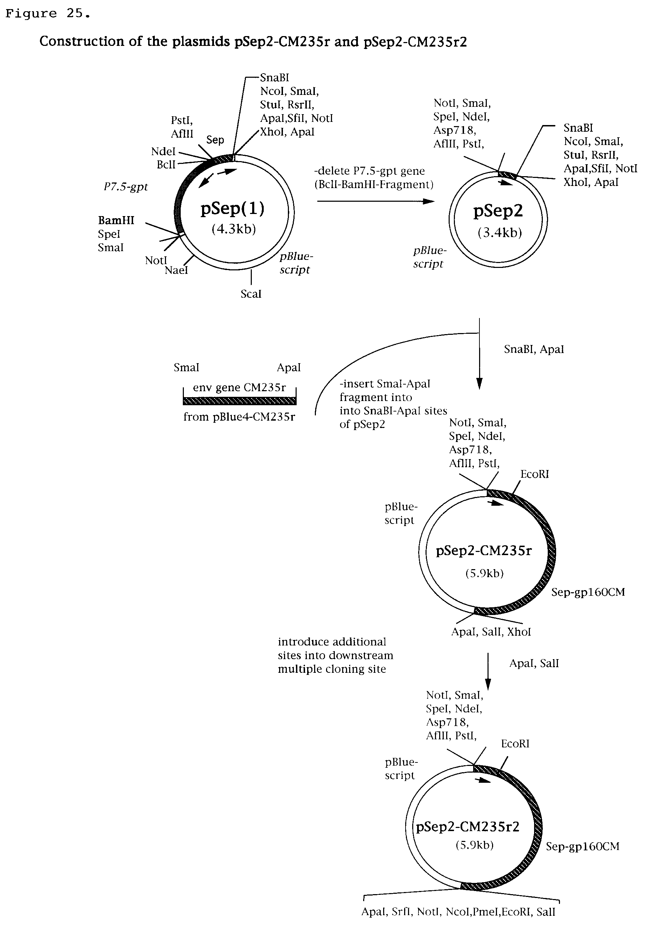

To construct mtk-CMenv the plasmid pSep2-CM235r2 (Figure 25) is inserted into the unique *Sma*I site of the virus mtkS as described in Example 12, or, alternatively, by recombination of the plasmids ptk-vA-CMenva and ptk-vA-CMenvb into MVA wildtype virus. The plasmid pSep(1) was cut with *Bcl*I and *BamH*I, the ends were filled in with Klenow Polymerase and the large fragment was religated. The resulting plasmid was designated pSep2. The SmaI-ApaI *env* gene fragment prepared out of the plasmid pBlue4-CM235r (described in Example 12) was inserted between the *SnaB*I and *Apa*I sites of plasmid pSep2 resulting in pSep2-CM235r. The plasmid pSep2-CM235r was cut with *Apa*I and *Sal*I and a linker consisting of the annealed oligonucleotides oSalApa1 and oSalApa2 (described in Example 12) was inserted between the two sites, resulting in the plasmid pSep2-CM235r2. The Sep-gp160CM gene cassette was inserted as a *Not*I fragment (obtained from the plasmid pSep2-CM235r2) into the plasmid ptk-vA, resulting in the orientational isomers ptk-vA-CMenva and ptk-vA-CMenvb.

The transient marker stabilisation procedure is carried out as described in Example 3. The resulting virus mtk-CMenv expresses HTV-1 gp160 and is further used for the construction of the *gagpol* and *env*-expressing virus m-gpe.

To construct the virus m-gpe primary chicken embryo fibroblasts are co-infected with 1 pfu/cell of the viruses mha-gagpol and mtk-CMenv. After three days of growth a viral crude stock is prepared of the cells and plaque purifications are done. The double recombinant viruses are identified by screening of single plaques by PCR and subsequent Western Blotting using the methods and antisera described in Examples 12 and 13. Viruses positive for gp160 and *gagpol* expression are further characterized by restriction analysis and Southern blotting.

### EXAMPLE 17 Immunization studies with m-gpe

The vaccinia virus m-gpe which is derived from the highly attenuated vaccine strain MVA expresses the HIV-1 genes gagpol and env. For immunization studies in chickens and rabbits a vaccine is prepared. The preparation of the virus vaccine is done as follows: The virus m-gpe and the control virus MVA wild-type are grown on chicken embryo fibroblasts and purified essentially as described by Joklik, Virol. 18: 9-18, 1962 with the exception, that the crude virus lysate is purified by one sucrose cushion.

Seroconversion experiments with 12 week-old SPF-chickens (Charles River, WIGA) are conducted to determine the immunizing properties of this virus strain. This particular seroconversion experiment is designed to examine the priming effects of the live virus m-gpe. Doses ranging from 10exp5 pfu to 10exp7 pfu per animal are used for immunization. The experimental design of the immunization study is outlined in Table 1. A second immunization is given after 3 weeks. A booster immunization of 50 ug per animal subunit gp160MN, or alternatively an inactivated HIV-1 whole virus vaccine, is given after another 3 weeks. Antibody development against gp160 and the gagpol gene products is analyzed by Western blotting and ELISA.

**Table 1**

| | | |
|---|---|---|
| Booster 2 and 3 consist of either 50ug gp160 in 0.5ml solution and alum as an adjuvant or 50ug of a whole virus vaccine. A group consists of three animals. Intravenous (iv) injections are given into the wing veins. | | |

| Vaccination of chickens with the virus m-gpe. | | |
|---|---|---|
| Group | First Immunization | Booster 1 |
| Group A iv | 0,5ml 10³ pfu m-gpe | 0,5ml 10⁵ pfu m-gpe |
| Group B iv | 0,5ml 10⁴ pfu m-gpe | 0,5ml 10⁶ pfu m-gpe |
| Group C iv | 0,5ml 10⁵ pfu m-gpe | 0,5ml 10⁷ pfu m-gpe |
| Group D iv | 0,5ml 10⁵ pfu MVA-WT | 0,5ml 10⁷ MVA-WT |
| Group E iv | 0,5ml PBS | 0,5ml PBS |
| Group F sc | 0,5ml 10⁴ pfu m-gpe | 0,5ml 10⁶ pfu m-gpe |
| Group G sc | 0,5ml 10⁵ pfu m-gpe | 0,5ml 10⁷ pfu f-aMN#31 |
| Group H sc | 0,5ml 10⁶ pfu m-gpe | 0,5ml 10⁸ pfu m-gpe |
| Group I sc | 0,5ml 10⁶ pfu MVA-WT | 0,5ml 10⁸ pfu MVA-WT |
| Group K sc | 0,5ml PBS | 0,5ml PBS |

In order to examine the capacity of the vaccine strains to prime humoral immune response in a non-avian species, rabbits are selected for study. The vaccination scheme is similar to the chicken experiment and is shown in Table 2. The animals are vaccinated twice with the live vector and then boosted with the gp160 subunit or the whole virus vaccine. Higher doses of the virus are used for immunization, because vaccinia MVA poorly replicates in rabbits.

The results demonstrate the use of m-gpe as a priming vehicle in animal systems such as chickens, in which MVA virus efficiently replicates, and in rabbits, in which vaccinia MVA only poorly replicates. Rabbits have long been used as models to evaluate human vaccines. The experiments therefore strongly suggest that also humans can be primed efficiently with the MVA derived recombinant viruses.

The immunizations of the rabbits are carried out essentially as shown in Table 2. In addition to the intravenous (i.v.) route, intramuscular (i.m.) and subcutaneous (s.c.) injections are given. Booster immunizations with gp160-subunit vaccine, and alternatively the HIV-1 whole virus vaccine are given at day 50. The HIV-1 MN-strain specific ELISA only is used to measure the immune response against gp160MN. The intramuscular injections also confirm the priming potential of m-gpe virus. The subcutaneous route also results in a measurable priming effect.

The gp160 specific ELISAS are done as follows: Microtiter plates are coated with purified gp160 at a concentration of 5ug/ml. After overnight incubation at 4°C, the plates are washed five times with PBS containing 0.05% Tween-20. Serum samples are serially diluted in PBS containing 0.5% Tween and 1% serum proteins, beginning with a 1:80 dilution. One hundred microliters of each sample are transferred to the coated plates. After an incubation of 1 hour at 37°C, the plates were washed five times with PBS-Tween solution and incubated for another hour with 100ul horseradish peroxidase-conjugated anti-IgG per well. After washing five times with PBS-Tween, 100µl of O-phenylenediamine dihydrochlorate was added to each well. The color reaction was stopped by the addition of 5M H₂SO₄ and the absorbance was measured at 495 nm with a microplate spectrophotometer. Whole Virus ELISA: The pooled sera were tested for whole HIV-1 virus with the whole virus kit of Behring Enzygnost as recommended by the manufacturer.

**TABLE 2**

| Vaccination of rabbits with the virus m-gpe. Booster 2 and 3 consist of either 50ug gp160 in 0.5ml solution and alum as an adjuvant or 50ug of a whole virus vaccine. A group consists of three animals. Intravenous (iv) injections are given into the ear veins; intramuscular injection (im) are given at two sites into the left and right thighs; subcutaneous injections are given at the same sites as im injections. | | |
|---|---|---|
| Group | First Immunization | Booster 1 |
| Group Z iv | 0,5ml 10⁶ pfu m-gpe | 0,5ml 10⁶ pfu m-gpe |
| Group Y iv | 0,5ml 10⁷ pfu m-gpe | 0,5ml 10⁷ pfu m-gpe |
| Group X iv | 0,5ml 10⁸ pfu m-gpe | 0,5ml 10⁸ pfu m-gpe |
| Group W iv | 0,5ml 10⁸ pfu MVA-WT | 0,5ml 10⁸ MVA-WT |
| Group V iv | 0,5ml PBS | 0,5ml PBS |
| Group U im | 0,5ml 10⁴ pfu m-gpe | 0,5ml 10⁶ pfu m-gpe |
| Group T im | 0,5ml 10⁵ pfu m-gpe | 0,5ml 10⁷ pfu f-aMN#31 |
| Group S im | 0,5ml 10⁶ pfu m-gpe | 0,5ml 10⁸ pfu m-gpe |
| Group R im | 0,5ml 10⁶ pfu MVA-WT | 0,5ml 10⁸ pfu MVA-WT |
| Group Q im | 0,5ml PBS | 0,5ml PBS |
| Group P sc | 0,5ml 10⁴ pfu m-gpe | 0,5ml 10⁶ pfu m-gpe |
| Group O sc | 0,5ml 10⁵ pfu m-gpe | 0,5ml 10⁷ pfu f-aMN#31 |
| Group N sc | 0,5ml 10⁶ pfu m-gpe | 0,5ml 10⁸ pfu m-gpe |
| Group M sc | 0,5ml 10⁶ pfu MVA-WT | 0,5ml 10⁸ pfu MVA-WT |
| Group L sc | 0,5ml PBS | 0,5ml PBS |

time schedule for vaccinations and blood samples
- Day -7: pre vaccination blood sample
- Day 0: first immunization
- Day 21: booster 1 and blood sample
- Day 35: booster 2 and blood sample
- Day 50: booster 3 and blood sample
- Day 79: blood sample
It is to be understood that the description, figures and specific examples, while indicating preferred embodiments of the invention, are given by way of illustration and are not intended to limit the present invention. Various changes and modifications within the spirit and scope of the invention will become apparent to those skilled in the art from the discussion and disclosure contained herein.

## Claims

1. A method for producing a recombinant eukaryotic cytoplasmic DNA virus, comprising the steps of
inserting a heterologous DNA construct into a eukaryotic cytoplasmic DNA virus genome, wherein said heterologous DNA construct comprises
(a) one or more segments of foreign DNA and (b) a double selection marker cassette which is flanked by (c) at least two direct repeat DNA sequences,
wherein said double selection marker cassette comprises a dominant selection marker gene and a color selection marker gene, and
wherein at least one foreign DNA segment does not occur within the DNA sequence bound by said direct repeat sequences and
transfecting cells with said eukaryotic cytoplasmic DNA virus containing said heterologous DNA construct and selecting a recombinant eukaryotic cytoplasmic DNA virus that does not contain said double selection marker cassette.

2. A method according to claim 1, wherein said recombinant eukaryotic cytoplasmic DNA virus is a recombinant poxvirus virus.

3. A method according to claim 2, wherein said recombinant poxvirus virus is a recombinant vaccinia virus or a recombinant avipoxvirus.

4. A method according to claim 3, wherein said vaccinia virus is derived from a vaccinia MVA strain and said avipoxvirus is derived from a fowlpox strain.

5. A method according to claim 1, wherein said inserting step is performed by *in vivo* homologous recombination or by direct molecular cloning.

6. A method according to claims 1-5, wherein said color selection marker gene is the *E. coli lacZ* gene and wherein said dominant selection marker gene is the *E. coli gpt* gene or the *E. coli hph* gene.

7. A method according to claims 1-6, wherein said foreign DNA encodes an antigen of a pathogen.

8. A method according to claim 7, wherein said antigen is from a pathogen selected from the group consisting of human immunodeficiency virus, human hepatitis virus and encephalitis virus.

9. A method according to claim 8, wherein said antigen gene is selected from the group consisting of HIV *env*, HIV *gag*, HIV *gagpol*, HIV *nef*, HBV preS1-S2-S-surface antigen gene, HBV pre-S2-S-surface antigen gene, HBV S-surface antigen gene, tick borne encephalitis *preM* gene and tick borne encephalitis *E* gene.

10. A method according to claims 1-9, wherein said foreign DNA further comprises a gene encoding an immunomodulator.

11. A method according to claim 10, wherein said immunomodulator is a cytokine.

12. A method according to claim 11, wherein said cytokine is interleukin 6 or interferon-γ.

13. A method according to any of the preceding claims 1-12, wherein the heterologous DNA construct is inserted into the vaccinia MVA hemagglutinin locus, the vaccinia MVA thymidine kinase locus, or the fowlpox thymidine kinase locus.

14. A method according to claim 13, wherein the foreign DNA further comprises a functional fowlpox thymidine kinase gene.

15. A recombinant eukaryotic cytoplasmic DNA virus producible by a method according to any of the preceding claims 1-14.

16. A recombinant eukaryotic cytoplasmic DNA virus according to claim 15, wherein said eukaryotic cytoplasmic DNA virus is an attenuated poxvirus.

17. A recombinant virus according to claim 15 or 16, wherein the virus is mhaS, mha-gag-pol, mtkS, mtk-gagpol, mtk-1Ag, mtk-prME, mtk-e/IFN, mtk-eIL6, m-gpe-IFN, m-gpe-IL6, m-gpe, f-tks, ftk-prME, ftk-gagpol.

18. A vaccine comprising an attenuated recombinant poxvirus according to any of the preceding claims 16 or 17 and a pharmaceutically acceptable carrier.

19. A vaccine according to claim 18, wherein said virus is live.

20. The use of a recombinant virus according to claim 16 or 17 for the vaccination of mammals.

21. A method of producing a vaccine, comprising the step of infecting cells with a recombinant eukaryotic cytoplasmic DNA virus according to claim 16 or 17.

22. A DNA molecule comprising:
(a) a multiple-cloning site and (b) a double selection marker cassette which is flanked by (c) at least two direct repeat DNA sequences, wherein the multiple-cloning site does not occur within the DNA sequence bound by said direct repeat DNA sequences, and
wherein said double selection marker cassette comprises a dominant selection marker gene and a color selection marker gene.

23. A DNA molecule according to claim 22, wherein said molecule is a plasmid selected from pvA(1), pvA(2), pHA-vA(2)a, pTK-vA, pTKm-vA(1), pTKm-vA(2).

24. A DNA molecule comprising:
(a) one or more segments of foreign DNA, and (b) a double selection marker cassette which is flanked by (c) at least two direct repeat DNA sequences, wherein at least one foreign DNA segment does not occur within the DNA sequence bounded by said direct repeat DNA sequences, and wherein said double selection marker cassette comprises a dominant selection marker gene and a color selection marker gene.

25. A DNA molecule according to claim 24, wherein said foreign DNA segment comprises a gene encoding an antigen of a pathogen.

26. A DNA molecule according to claim 25, wherein said antigen gene is from a pathogen selected from the group consisting of human hepatitis B virus, HIV, and tick borne encephalitis virus.

27. A DNA molecule according to claim 26, wherein said antigen gene is selected from the group consisting of HIV *env*, HIV gag, HIV *gagpol*, HIV *nef*, HBV preS1-S2-S-surface antigen gene, HBV pre-S2-S-surface antigen gene, HBV S-surface antigen gene, tick borne encephalitis *preM* gene and tick borne encephalitis *E* gene.

28. A DNA molecule according to any of the preceding claims 24 to 27, wherein said foreign DNA segment further comprises a gene encoding an immunomodulator.

29. A DNA molecule according to claim 28, wherein said immunomodulator is a cytokine.

30. A DNA molecule according to claim 29, wherein said cytokine is interleukin 6 or interferon-γ.

31. A DNA molecule according to claim 30, wherein said color selection marker gene is the *E. coli lacZ* gene and said dominant selection marker gene is either the *E. coli hph* gene or the *E. coli gpt* gene.

32. A DNA molecule according to claim 31, wherein the foreign DNA comprises a functional fowlpox thymidine kinase gene.


SEQUENCE LISTING

(1) GENERAL INFORMATION:

(i) APPLICANT:
(A) NAME: Immuno Aktiengesellchaft
(B) STREET: Industriestraße 67
(C) CITY: Wien
(D) STATE: Austria
(E) COUNTRY: Austria
(F) POSTAL CODE (ZIP): 1221

(ii) TITLE OF INVENTION: Improved Recombinant Eukaryotic Cytoplasmic
Viruses, Method for their Production and their Use as Vaccines

(iii) NUMBER OF SEQUENCES: 42

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 45 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 1:

GGGTAAAAGT TGAAAATATA TTTCTATGCT ATAAATAAGT TAACG 45

(2) INFORMATION FOR SEQ ID NO: 2:

(i) SEQUENCE CHARACTERISTICS:
(A) LENGTH: 49 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 2:

AATTCGTTAA CTTATTTATA GCATAGAAAT ATATTTTCAA CTTTTACCC 49

(2) INFORMATION FOR SEQ ID NO: 3:

(i) SEQUENCE CHARACTERISTICS:
(A) LENGTH: 39 base pairs
(B) TYPE: nucleic acid
(C) STRANDEDNESS: single

(D) TOPOLOGY: linear

(ii) MOLECULE TYPE: DNA (genomic)

(xi) SEQUENCE DESCRIPTION: SEQ ID NO: 3:

ATCATATGAG GATCCATGAG CGAAAAATAC ATCGTCACC 39

(2) INFORMATION FOR SEQ ID NO: 4:

(i) SEQUENCE CHARACTERISTICS:
(A) LENGTH: 43 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 4:

ATCCCGGGAC GTCATAAAAA GATTAGCGAC CGGAGATTGG CGG 43

(2) INFORMATION FOR SEQ ID NO: 5:

(i) SEQUENCE CHARACTERISTICS:
(A) LENGTH: 46 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 5:

TATGTAAAAG TTGAAAATAT ATTTCTATGC TATAAATAAG TTAACA 46

(2) INFORMATION FOR SEQ ID NO: 6:

(i) SEQUENCE CHARACTERISTICS:
(A) LENGTH: 48 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 6:

GATCTGTTAA CTTATTTATA GCATAGAAAT ATATTTTCAA CTTTTACA 48

(2) INFORMATION FOR SEQ ID NO: 7:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 7:

AGGCCTGATA TCGTCGACGT TAACTGATCA AGATCT 36

(2) INFORMATION FOR SEQ ID NO: 8:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 8:

AGATCTTGAT CAGTTAACGT CGACGATATC AGGCCT 36

(2) INFORMATION FOR SEQ ID NO: 9:

(i) SEQUENCE CHARACTERISTICS:
(A) LENGTH: 48 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 9:

TCGAAATAAC TTCGTATAAT GTATGCTATA CGAAGTTATA GCGCTGTT 48

(2) INFORMATION FOR SEQ ID NO: 10:

(i) SEQUENCE CHARACTERISTICS:
(A) LENGTH: 44 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 10:

AACAGCGCTA TAACTTCGTA TAGCATACAT TATACGAAGT TATT 44

(2) INFORMATION FOR SEQ ID NO: 11:

(i) SEQUENCE CHARACTERISTICS:
(A) LENGTH: 62 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 11:

GTGGCCAGTT AACGGATCCT TTAAAAAGCT TGTCGACTTT AAATACGTAA GATCTTGGCC 60

AA 62

(2) INFORMATION FOR SEQ ID NO: 12:

(i) SEQUENCE CHARACTERISTICS:
(A) LENGTH: 62 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 12:

TTGGCCAAGA TCTTACGTAT TTAAAGTCGA CAAGCTTTTT AAAGGATCCG TTAACTGGCC 60

AC 62

(2) INFORMATION FOR SEQ ID NO: 13:

(i) SEQUENCE CHARACTERISTICS:
(A) LENGTH: 63 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 13:

AGTTTAAACG GCGCGCCCGG GCTCGAGAGG CCTCTGCAGA TGCATCCATG GGGATCCGAA 60

TTC 63

(2) INFORMATION FOR SEQ ID NO: 14:

(i) SEQUENCE CHARACTERISTICS:
(A) LENGTH: 63 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 14:

GAATTCGGAT CCCCATGGAT GCATCTGCAG AGGCCTCTCG AGCCCGGGCG CGCCGTTTAA 60

ACT 63

(2) INFORMATION FOR SEQ ID NO: 15:

(i) SEQUENCE CHARACTERISTICS:
(A) LENGTH: 48 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 15:

GATCCTACGT ATCTAGAATT AATTAATGGC CAATTTAAAT GCCCGGGA 48

(2) INFORMATION FOR SEQ ID NO: 16:

(i) SEQUENCE CHARACTERISTICS:
(A) LENGTH: 48 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 16:

AATTTCCCGG GCATTTAAAT TGGCCATTAA TTAATTCTAG ATACGTAG 48

(2) INFORMATION FOR SEQ ID NO: 17:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 17:

GCCCGGGCAG CGCTGCGGCC GCAGGCCTGC CCGGG 35

(2) INFORMATION FOR SEQ ID NO: 18:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 18:

GCCCGGGCAG GCCTGCGGCC GCAGCGCTGC CCGGGC 36

(2) INFORMATION FOR SEQ ID NO: 19:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 19:

GGTAGGGATA ACAGGGTAAT AA 22

(2) INFORMATION FOR SEQ ID NO: 20:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 20:

TTATTACCCT GTTATCCCTA CC 22

(2) INFORMATION FOR SEQ ID NO: 21:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 21:

GGATCTACAC ATTCACCAGA AACTA 25

(2) INFORMATION FOR SEQ ID NO: 22:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 22:

CCGGAGTCTC GTCTGTTGTG GATTCTCCA 29

(2) INFORMATION FOR SEQ ID NO: 23:

(i) SEQUENCE CHARACTERISTICS:
(A) LENGTH: 41 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 23:

CTGGCTCTGC CTTGGAAACA TGAGGGCGCG CAAAACTGGA C 41

(2) INFORMATION FOR SEQ ID NO: 24:

(i) SEQUENCE CHARACTERISTICS:
(A) LENGTH: 45 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 24:

CTGGATCCAG TTTTGCGCGC CCTCATGTTT CCAAGGCAGA GCCAG 45

(2) INFORMATION FOR SEQ ID NO: 25:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 25:

CTACCATGGC GTGGATGAGC TGGTTGCTGG TC 32

(2) INFORMATION FOR SEQ ID NO: 26:

(i) SEQUENCE CHARACTERISTICS:
(A) LENGTH: 45 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 26:

CAGTCGACCA TGGACTAGTT AACTACGCCC CCACTCCAAG GGTCA 45

(2) INFORMATION FOR SEQ ID NO: 27:

(i) SEQUENCE CHARACTERISTICS:
(A) LENGTH: 84 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 27:

TATGAGATCT AAAAATTGAA ATTTTATTTT TTTTTTTTGG AATATAAATA AGGCCTCCAT 60

GGCCATAATT AATTAATTTT TCTG 84

(2) INFORMATION FOR SEQ ID NO: 28:

(i) SEQUENCE CHARACTERISTICS:
(A) LENGTH: 86 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 28:

TCGACAGAAA AATTAATTAA TTATGGCCAT GGAGGCCTTA TTTATATTCC AAAAAAAAAA 60

AATAAAATTT CAATTTTTAG ATCTCA 86

(2) INFORMATION FOR SEQ ID NO: 29:

(i) SEQUENCE CHARACTERISTICS:
(A) LENGTH: 68 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 29:

CGATGCGGCC GCGCCCGGGC GCGGCCGCTA GGGATAACAG GGTAATGGCG CGCCGGCCAT 60

ATAGGCCA 68

(2) INFORMATION FOR SEQ ID NO: 30:

(i) SEQUENCE CHARACTERISTICS:
(A) LENGTH: 70 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 30:

GTACTGGCCT ATATGGCCGG CGCGCCATTA CCCTGTTATC CCTAGCGGCC GCGCCCGGGC 60

GCGGCCGCAT 70

(2) INFORMATION FOR SEQ ID NO: 31:

(i) SEQUENCE CHARACTERISTICS:
(A) LENGTH: 66 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 31:

ATTCTCCACA TACCTAGAAG AATCAGACAG GGCTTGGAAA GGGCTTTGCT ATAAGTCGAC 60

CATGGG 66

(2) INFORMATION FOR SEQ ID NO: 32:

(i) SEQUENCE CHARACTERISTICS:
(A) LENGTH: 66 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 32:

CCCATGGTCG ACTTATAGCA AAGCCCTTTC CAAGCCCTGT CTGATTCTTC TAGGTATGTG 60

GAGAAT 66

(2) INFORMATION FOR SEQ ID NO: 33:

(i) SEQUENCE CHARACTERISTICS:
(A) LENGTH: 46 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 33:

TCGACGAATT CGTTTAAACC CATGGGCGGC CGCGCCCGGG CGGGCC 46

(2) INFORMATION FOR SEQ ID NO: 34:

(i) SEQUENCE CHARACTERISTICS:
(A) LENGTH: 38 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 34:

CGCCCGGGCG CGGCCGCCCA TGGGTTTAAA CGAATTCG 38

(2) INFORMATION FOR SEQ ID NO: 35:

(i) SEQUENCE CHARACTERISTICS:
(A) LENGTH: 78 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 35:

CATGAAATAT ACAAGTTATA TCTTGGCTTT TCAGCTCTGC ATCGTTTTGG GTTCTCTTGG 60

CTGTTACTGC CAGGACCC 78

(2) INFORMATION FOR SEQ ID NO: 36:

(i) SEQUENCE CHARACTERISTICS:
(A) LENGTH: 78 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 36:

GTACGGGTCC TGGCAGTAAC AGCCAAGAGA ACCCAAAACG ATGCAGAGCT GAAAAGCCAA 60

GATATAACTT GTATATTT 78

(2) INFORMATION FOR SEQ ID NO: 37:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 37:

AAGGCCTCAT GAAATATACA AGTTATATCT TGGCT 35

(2) INFORMATION FOR SEQ ID NO: 38:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 38:

AAGGCCTGCA TGCAGGACCC GTACGTA 27

(2) INFORMATION FOR SEQ ID NO: 39:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 39:

GTTTTCCCAG TCACGACGTT GGTGTGGAAT TGTGAGCGGA 40

(2) INFORMATION FOR SEQ ID NO: 40:

(i) SEQUENCE CHARACTERISTICS:
(A) LENGTH: 115 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 40:

AAGGCCTCAT GAACTCCTTC TCCACAAGCG CCTTCGGTCC AGTTGCCTTC TCCCTGGGGC 60

TGCTCCTGGT GTTGCCTGCT GCCTTCCCTG CCCCAGTACC CCCAGGAGAA GATTC 115

(2) INFORMATION FOR SEQ ID NO: 41:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 41:

GTTTTCCCAG TCACGACGTT G 21

(2) INFORMATION FOR SEQ ID NO: 42:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 42:

AAGGCCTGCA TGCCGGTACC CCCAGGA 27